# EUROPEAN PATENT APPLICATION

(11) **EP 2 261 211 A1**
(43) Date of publication of application: **15.12.2010**
(21) Application number: 09290377.2
(22) Date of filing: 20.05.2009
(51) Int. Cl.: C07D 213/82, C07D 405/12, A61K 31/33, A61P 29/00

(54) **1,4-dihydropyridine derivatives and their uses**

(71) Applicant: Université de Lille 2 Droit et Santé, 59800 Lille (FR)
(72) Inventor: Millet, Regis, 62240 Harnes (FR); El Bakali, Jamal, 59000 Lille (FR); Chavatte, Philippe, 59147 Gondecourt (FR); Renault, Nicolas, 59120 Loos (FR); Lambert, Didier, 1332 Genval (BE); Muccioli, Giulo, 1560 Hoeilaart (BE); Body-Malapel, Mathilde, 59133 Phalempin (FR); Desreumaux, Pierre, 59700 Marcq en Barouel (FR)
(74) Representative: Noel, Chantal Odile

(57) **Abstract**

The present invention relates to 1,4-dihydropyridine derivatives of the formula (I) and their uses in the treatment and/or prevention of diseases and disorders directly or indirectly associated with the modification (increase or decrease) of the activity of the cannabinoid receptor 2 (CB2).

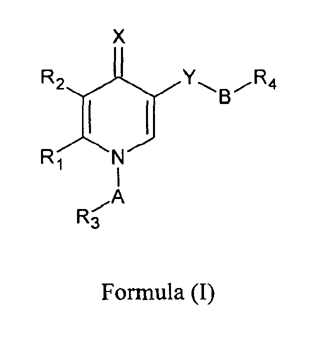

## Description

The present invention relates to 1,4-dihydropyridine derivatives of the formula (I) and their uses in the treatment of diseases associated with a modification of the activity of the cannabinoid receptor 2 (CB2).

Cannabinoids are bioactive lipids found in the *Cannabis sativa* (marijuana) plant. In addition to their well-documented effects on mood, cannabinoids (often extracted from marijuana) have been used to treat nausea, pain, migraine, epilepsy, glaucoma, hypertension, cachexia and pain associated with childbirth. Botanical preparations of *Cannabis sativa* (Indian hemp) have been widely used in the past to treat a variety of disorders including those affecting the digestive tract. In 1964, Δ9-tetrahydrocannabinol (Δ9-THC) was isolated (1), and was later shown to be responsible for many of the pharmacological actions of *Cannabis* preparations. The understanding of the mechanism by which marijuana exerts its pharmacological action has seen considerable progress following the discovery in the early 1990s of specific membrane, G-protein-coupled receptors for Δ9-THC, namely CB1 receptors (2), expressed by central and peripheral nerves (including the enteric nervous system), and CB2 receptors (3), which occur mainly in immune cells.

The discovery of these receptors has led to the demonstration that there are endogenous agonists for these receptors. The best known are anandamide, 2-arachidonylglycerol (2-AG) (nonselective cannabinoid receptor agonists), noladin ether (CB1 receptor agonist) and virodhamine (CB1 receptor antagonist/CB2 receptor agonist). When released, anandamide and 2-AG are removed from extracellular compartments by a carrier-mediated reuptake process, and once within the cell, both endocannabinoids are hydrolyzed by the enzyme fatty acid amide hydrolase (FAAH) (4). In addition to the two cannabinoid receptors, anandamide (5) and 2-AG (6) (both detected in the gut) can also activate vanilloid receptors, the molecular target for the pungent plant compound capsaicin (7).

The psychotropic effects caused by Δ9-THC and other nonselective CB agonists are mediated by central CB1 receptors. These CB1 receptor-mediated effects, such as euphoria, sedation, hypothermia, catalepsy, and anxiety, have limited the development and clinical utility of CB1 receptor-interacting ligands.

Recent studies have demonstrated that CB2 modulators are analgesics in preclinical models (8) of inflammatory and neuropathic pain without causing the adverse side effects associated with CB1 receptor activation.

Altered visceral perception and pain are commonly found in patients with inflammatory bowel disease (IBD) and may persist in those in remission from this condition. Pain is also one of the defining features of irritable bowel syndrome (IBS) and other functional bowel disorders, which are conditions of altered bowel function and pain that are not associated with physical abnormalities of the gut wall. Recent evidence has shed light on a role for CB2 receptors in pain states, particularly those originating from the gut (9, 10). Therefore, compounds that selectively target CB2 receptors are an attractive approach for the development of novel analgesics in gut pain.

The strong expression of CB2 receptor in tissues and cells of immune system (11), as well as the enhancement of the expression of this receptor after inflammatory insults (12) suggests that CB2 selective ligands might be effective in modulating inflammation. These observations were confirmed by the lack of immunomodulation induced by cannabinoids in CB2 knockout mice (13). The CB2 receptor exerts a critical role in the immune system by modulating the release of cytokines (14), and the migration of immune cells (15). Besides, recent studies have demonstrated that CB2 receptor ligands have immunomodulatory and anti-inflammatory properties in the gut (for a recent review see 16). Therefore, compounds that interact with CB2 receptors offer a unique pharmacotherapy for the treatment of immune and inflammatory disorders or diseases, especially Inflammatory Bowel Disease (IBD) and also for the treatment of some intestinal disorders like Irritable Bowel Syndrome (IBS).

The Inventors have now found that, unexpectedly, 1,4-dihydropyridine derivatives of formula (I) below are useful as CB2 receptor modulators.

Thus, the invention relates to the compounds of formula (I) below: 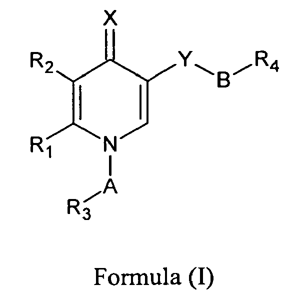 or pharmaceutically acceptable salts or solvates thereof,
wherein:
X represents oxygen or sulphur;
Y represents C=O, CONH, NHCO, NHCONH, NHSO₂-CO, NH(CO)O, NR₉; R₉ representing a hydrogen atom or C₁₋₃ alkyl;
A represents (CR₇R₈)ₘ; R₇ and R₈, identical or different, representing an hydrogen atom or C₁₋₃ alkyl; and m representing an integer equal to 0, 1, 2, 3 or 4; and when m=0, A represents a single bond;
B represents (CR₇R₈)ₙ; R₇ and R₈ being as defined above and n representing an integer equal to 0, 1, 2, 3 or 4; and when n=0, B represents a single bond;
R₁ and R₂ are identical or different and represent independently a hydrogen atom, a halogen atom, CN, CF₃, OCF₃, OCHF₂, an alkyl, an alkoxy, (CH₃)₃C, a cycloalkyl, an optionally substituted 5 to 10 membered aryl, an optionally substituted 5 to 7 membered monocyclic heteroaryl containing 1 to 3 heteroatoms selected from O, N, S, or an optionally substituted 9 to 10 membered fused bicyclic heteroaryl containing 1 to 3 heteroatoms selected from O, N and S; and
R₃ and R₄ are identical or different and represent independently a hydrogen atom, an alkyl, an alkoxy, an optionally substituted tetrahydropyranyl, morpholinyl or cycloalkyl, an optionally substituted 6 to 10 membered aryl, an optionally substituted 5 to 7 membered monocyclic heteroaryl containing 1 to 3 heteroatoms selected from O, N, S, or an optionally substituted 9 to 10 membered fused bicyclic heteroaryl containing 1 to 3 heteroatoms selected from O, N and S.

As used herein, the term "halogen" means F, Cl, Br and I. Unless indicated otherwise, halogenated substituents preferably carry one, two, three, four or five identical or different halogen atoms.

As used herein, the term "alkyl" refers to a straight or branched hydrocarbon chain and may comprise 1 to 20, preferably 1 to 6, more preferably 1 to 4 carbon atoms, straight or branched. Examples of alkyls are methyl, ethyl, propyl, 1-methylethyl, butyl, 1-methylpropyl, 2-methylpropyl, 1, 1-dimethylethyl. examples of c1-c6 alkyls include methyl, ethyl, propyl, 1-methylethyl, butyl, 1-methylpropyl, 2-methylpropyl, 1, 1-dimethylethyl, n-pentyl, 1-methylbutyl, 2-methylbutyl, 3-methylbutyl, 2,2-dimethylpropyl, 1-ethylpropyl, hexyl, 1, 1-dimethylpropyl, 1,2-dimethylpropyl, 1-methylpentyl, 2-methylpentyl, 3-methylpentyl, 4-methylpentyl, 1, 1-dimethylbutyl, 1, 2-dimethylbutyl, 1, 3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1-ethyl-1-methylpropyl or 1-ethyl-3-methylpropyl, n-pentyl, 1-methylbutyl, 2-methylbutyl, 3-methylbutyl, 2,2-dimethylpropyl, 1-ethylpropyl, hexyl, 1, 1-dimethylpropyl, 1,2-dimethylpropyl, 1-methylpentyl, 2-methylpentyl, 3-methylpentyl, 4-methylpentyl, 1, 1-dimethylbutyl, 1, 2-dimethylbutyl, 1, 3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1-ethyl-1-methylpropyl and 1-ethyl-3-methylpropyl.

Preferably, the alkyl is selected from the group consisting of methyl (Me), ethyl (Et), n-propyl, i-propyl, butyl, pentyl, *tert*-butyl, n-hexyl , i-hexyl, 1-, 2-, 3-methylbutyl and trimethylpropyl.

As used herein, the term "alkoxy" (when used as a group or as part of a group) refers to an alkyl ether radical, wherein the term "alkyl" is defined above, for exemple methoxy, ethoxy, propoxy, 1-methylethoxy, butoxy, 1-methyl- propoxy, 2-methylpropoxy, 1, 1-dimethylethoxy, pentoxy, 1-methylbutoxy, 2-methylbutoxy, 3-methylbutoxy, 1, 1-dimethylpropoxy, 1, 2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1, 1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy, preferably methoxy, ethoxy, n-propoxy, i-propoxy, n-pentoxy and i-pentoxy.

As used herein, the term "cycloalkyl" means a monocyclic bicyclic or tricyclic nonaromatic saturated hydroarbon radical having 3 to 10 carbon atoms, such as 3 to 8 carbon atoms, for example, 3 to 6 carbons atoms. Suitable cycloalkyl groups include, but are not limited to, cyclopropyl, cyclobutyl, cyclopentyl, cyclohexyl, cycloheptyl, cyclooctyl, norbornyl, 1-decalin, adamant-1-yl, adamant-2-yl. Other suitable cycloalkyl groups include, but are not limited to, spiropentyl, bicyclo[2.1.0]pentyl, bicyclo[3.1.0]hexyl, spiro[2.4]heptyl, spiro[3.3]heptyl, bicyclo[4.2.0]octyl, and spiro[3.5]nonyl. Preferred cycloalkyl groups include cyclopropyl, cyclohexyl, adamant-1-yl and adamant-2-yl.

The term "aryl" as used herein refers to a C₅₋₁₀ monocyclic or bicyclic hydrocarbon ring wherein at least one ring is aromatic. Examples of such groups include phenyl and naphthyl.

The term "heteroaryl", unless stated otherwise, is intended to mean a 5 to 7 membered monocyclic aromatic or a fused 9 to 10 membered bicyclic aromatic ring containing 1, 2 or 3 heteroatoms, identical or different selected from the group consisting of oxygen, nitrogen and sulfur. Preferred examples of such monocyclic aromatic rings include thienyl, furanyl, pyrrolyl, triazolyl, imidazolyl, oxazolyl, thiazolyl, oxadiazolyl, isothiazolyl, isoxazolyl, thiadiazolyl, pyrazolyl, pyrimidyl, pyridazinyl, pyrazinyl and pyridyl. Suitable examples of such fused bicyclic aromatic rings include quinolinyl, isoquinolinyl, quinazolinyl, quinoxalinyl, cinnolinyl, naphthyridinyl, indolyl, indazolyl, pyrrolopyridinyl, benzofuranyl, benzothienyl, benzimidazolyl, benzoxazolyl, benzisoxazolyl, benzothiazolyl, benzisothiazolyl, benzoxadiazolyl and benzothiadiazolyl.

All phenyl rings or heterocyclyl radicals and all phenyl components or heterocyclyl components are, unless stated otherwise, preferably unsubstituted, or they carry one, two or three halogen atoms and/or one or two methyl, trifluoroethyl, ethoxy or trifluoromethoxy substituents.

According to preferred embodiments of the invention:
- When R₁ or R₂ represent an optionally substituted 5 to 10 membered aryl, an optionally substituted 5 to 7 membered monocyclic heteroaryl containing 1 to 3 heteroatoms selected from O, N, S, or an optionally substituted 9 to 10 membered fused bicyclic heteroaryl containing 1 to 3 heteroatoms selected from O, N and S, they may be substituted by one or more substituents which may be the same or different, selected from the group consisting of an halogen, hydroxyl, CN, nitro, NR₇R₈, CONR₇R₈, CF₃, OCF₃, OCHF₂, an alkyl, an alkoxy, COC₁₋₆ alkyl, COC₁₋₆ alkoxy, NHCOC₁₋₆ alkyl and COOH;
- When R₃ or R₄, represent an optionally substituted tetrahydropyranyl, morpholinyl, cycloalkyl, they may be substituted by one or more substituents which may be, identical or different, selected from the group consisting of halogen, hydroxyl, CN, nitro, NR₇R₈, CONR₇R₈, CF₃, OCF₃, OCHF₂, alkyl, alkoxy, COC₁₋₄ alkyl, COC₁₋₄ alkoxy, NHCOC₁₋₆ alkyl and COOH; and
- When R₃ or R₄, represent an optionally substituted 6 to 10 membered aryl, an optionally substituted 5 to 7 membered monocyclic heteroaryl containing 1 to 3 heteroatoms selected from O, N, S, or an optionally substituted 9 to 10 membered fused bicyclic heteroaryl containing 1 to 3 heteroatoms selected from O, N and S, they may be substituted by one or more substituents which may be, identical or different, selected from the group consisting of an halogen, hydroxyl, CN, nitro, NR₇R₈, CONR₇R₈, CF₃, OCF₃, OCHF₂, an alkyl, an alkoxy, COC₁₋₆ alkyl, COC₁₋₆ alkoxy, NHCOC₁₋₆ alkyl and COOH.

According to another preferred embodiment of the invention, the compounds of formula (I) have a binding affinity to the CB2 receptor, measured by the inhibition constant Kᵢ = IC₅₀/(1+L/K_{d}), said Kᵢ being of less than 1500 nM, preferably of less than 500 nM and more preferably of less than 100 nM.

Thus, the binding affinity is expressed by means of the inhibition constant Kᵢ; it could also be expressed by the half-maximal inhibition constant IC₅₀. The inhibition constant Kᵢ and the half-maximal inhibition constant IC₅₀ are measured in competitive binding experiments.

For measuring the inhibition constant Kᵢ of CB2 receptor ligands, [³H]-CP-55,9540 can be used as a radioligand for the CB2 receptor in competitive binding experiments.

The Kᵢ values are determined from IC₅₀ values, using the Cheng-Prusoff equation: Kᵢ = IC₅₀/(1+L/K_{d}).

IC₅₀ and EC₅₀ values are determined by non-linear regression analysis performed using the GraphPad prism 4.0 program (GraphPad Software, San Diego) (19, 20).

Binding is understood as meaning any molecular interaction between the ligand and the receptor. As a rule, these are conventional interactions, which include electrostatic attraction, hydrogen bonding, hydrophobic bonds, van-der-Waals forces, metal complex-like coordinative bonds and covalent bonds.

Specific binding affinity to the CB2 receptor is given when the Kᵢ value of the compound of the formula (I) at the cloned human CB2 receptor is at least 5, more preferably at least 10, more preferably at least 15 and most preferably 20 times the Kᵢ value at the cloned human CB1 receptor.

Some of the instant compounds have a binding affinity of more than 500 nM.

They are listed in the Table below:

**Table I. Compounds of Formula I with a binding affinity > 500 nM.**

| **Compound No.** | X | Y | R₁ | R₂ | R₃ | R₄ |
|---|---|---|---|---|---|---|
| **8** | O | 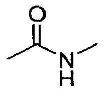 | 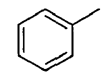 | H | 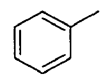 | 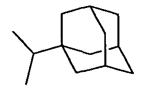 |
| **22** | O | 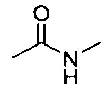 | 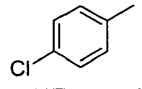 | H | C₅H₁₁ | 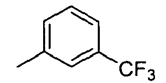 |

According to another preferred embodiment of the invention, when the compounds of the formula (I) have a binding affinity to the CB2 receptor measured by the inhibition constant Kᵢ of less than 500 nM:
- m=0, A represents a single bond and R₃ represents an alkyl having 1 to 6 carbon atoms, more preferably butyl, pentyl or hexyl; and/or
- the radical R₂ represents preferably an hydrogen atom, a halogen atom, an alkyl having 1 to 6 carbon atoms, a cycloalkyl or an aryl, more preferably an hydrogen atom, a iodine atom, cyclopropyl, *p*-tolyl, meta-cyanophenyl or phenyl; and/or
- n=0; B represents a single bond and R₄ represents an alkyl, a cycloalkyl, an aryl or an heteroaryl, more preferably t-butyl, cyclohexyl, adamantyl, adamantylethyl, dimethyladamantyl or phenyl, trifluoromethylphenyl, tetrahydronaphtyl or piperidin and the radical Y represents CONH, NHCO, NHCONH or NH(CO)O.

Preferable radicals Y and R₄ (B representing a single bond) are represented in Table I below.

**Table II: Preferred radicals Y and R₄:**

| **Y** | **R₄** |
|---|---|
| CONH | H |
| CONH | CH₃ |
| CONH | C₂H₅ |
| CONH | n-C₃H₇ |
| CONH | i-C₃H₇ |
| CONH | n-C₄H₉ |
| CONH | i-C₄H₉ |
| CONH | s-C₄H₉ |
| CONH | t-C₄H₉ |
| CONH | Tetrahydropyranyl |
| CONH | Morpholinyl |
| CONH | Cyclopropyl |
| CONH | Cyclopentyl |
| CONH | Cyclohexyl |
| CONH | Adamantyl |
| CONH | 1-(1-Adamantyl)methyl |
| CONH | 1-(1-Adamantyl)ethyl |
| CONH | 1-(3,5-Dimethyl)adamantyl |
| CONH | Phenyl |
| CONH | 3(trifluoromethyl)phenyl |
| CONH | 1,2,3,4-tetrahydronaphthyl |
| CONH | piperidin-1-yl |
| NHCO | H |
| NHCO | CH₃ |
| NHCO | C₂H₅ |
| NHCO | n-C₃H₇ |
| NHCO | i-C₃H₇ |
| NHCO | n-C₄H₉ |
| NHCO | i-C₄H₉ |
| NHCO | s-C₄H₉ |
| NHCO | t-C₄H₉ |
| NHCO | Tetrahydropyranyl |
| NHCO | Morpholinyl |
| NHCO | Cyclopropyl |
| NHCO | Cyclopentyl |
| NHCO | Cyclohexyl |
| NHCO | Adamantyl |
| NHCO | 1-(1-Adamantyl)methyl |
| NHCO | 1-(1-Adamantyl)ethyl |
| NHCO | 1-(3,5-Dimethyl)adamantyl |
| NHCO | Phenyl |
| NHCO | 3(trifluoromethyl)phenyl |
| NHCO | 1,2,3,4-tetrahydronaphthyl |
| NHCO | piperidin-1-yl |
| NHCONH | H |
| NHCONH | CH₃ |
| NHCONH | C₂H₅ |
| NHCONH | n-C₃H₇ |
| NHCONH | i-C₃H₇ |
| NHCONH | n-C₄H₉ |
| NHCONH | i-C₄H₉ |
| NHCONH | s-C₄H₉ |
| NHCONH | t-C₄H₉ |
| NHCONH | Tetrahydropyranyl |
| NHCONH | Morpholinyl |
| NHCONH | Cyclopropyl |
| NHCONH | Cyclopentyl |
| NHCONH | Cyclohexyl |
| NHCONH | Adamantyl |
| NHCONH | 1-(1-Adamantyl)methyl |
| NHCONH | 1-(1-Adamantyl)ethyl |
| NHCONH | 1-(3,5-Dimethyl)adamantyl |
| NHCONH | Phenyl |
| NHCONH | 3(trifluoromethyl)phenyl |
| NHCONH | 1,2,3,4-tetrahydronaphthyl |
| NHCONH | piperidin-1-yl |

Particularly preferred compounds of formula (I) with a binding affinity for CB2 receptors measured by the inhibition constant Kᵢ of less than 500 nM are those wherein R₂ represents a hydrogen atom or a iodine atom, A and B represent a single bond and X, Y, R₁, R₃ and R₄ are represented in Table III below.

**Table III: Particularly preferred compounds of formula (I)**

| **Compound No.** | **X** | **Y** | **R₁** | **R₂** | **R₃** | **R₄** |
|---|---|---|---|---|---|---|
| **6** | O | 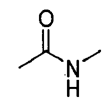 | 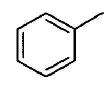 | H | C₂H₅ | 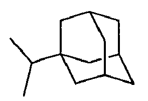 |
| **7** | O | 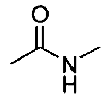 | 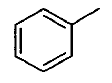 | H | C₅H₁₁ | 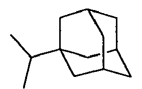 |
| **9** | O | 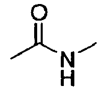 | 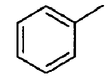 | H | C₅H₁₁ | 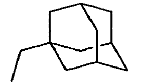 |
| **10** | O | 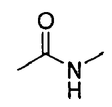 | 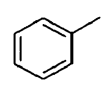 | H | C₅H₁₁ | 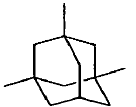 |
| **11** | O | 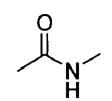 | 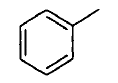 | H | C₄H₉ | 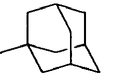 |
| **12** | O | 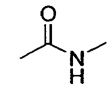 | 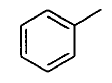 | H | C₅H₁₁ | 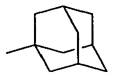 |
| **13** | O | 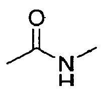 | 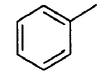 | H | C₆H₁₃ | 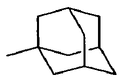 |
| **14** | O | 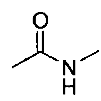 | 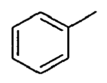 | H | C₅H₁₁ | 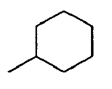 |
| **15** | O | 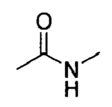 | 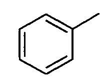 | H | C₅H₁₁ | 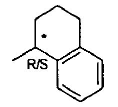 |
| **16** | O | 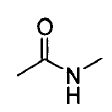 | 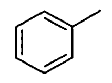 | H | C₅H₁₁ | 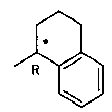 |
| **17** | O | 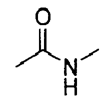 | 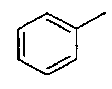 | H | C₅H₁₁ | 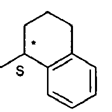 |
| **18** | O | 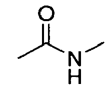 | 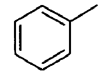 | H | C₅H₁₁ | 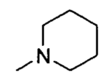 |
| **19** | O | 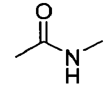 | 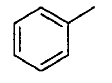 | H | C₅H₁₁ | 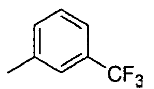 |
| **20** | O | 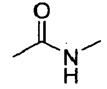 | 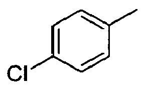 | H | C₅H₁₁ | 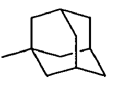 |
| **21** | O | 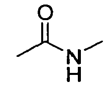 | 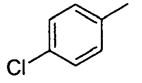 | H | C₅H₁₁ | 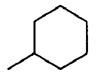 |
| **23** | O | 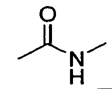 | t-butyl | H | C₅H₁₁ | 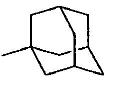 |
| **24** | O | 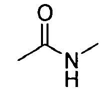 | t-butyl | H | C₅H₁₁ | 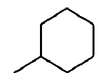 |
| **25** | S | 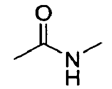 | 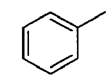 | H | C₅H₁₁ | 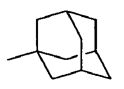 |
| **26** | S | 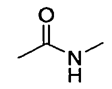 | 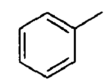 | H | C₅H₁₁ | 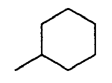 |
| **27** | O | 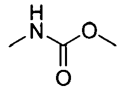 | 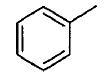 | H | C₅H₁₁ | 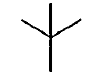 |
| **29** | O | 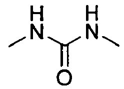 | 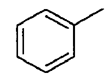 | H | C₅H₁₁ | 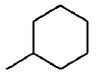 |
| **30** | O | 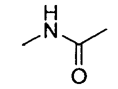 | 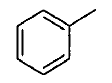 | H | C₅H₁₁ | 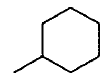 |
| **33** | O | 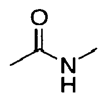 | CH₃ | H | C₅H₁₁ | 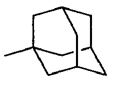 |
| **35** | O | 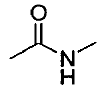 | CH₃ | I | C₅H₁₁ | 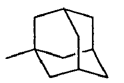 |

Among the compounds of Tables I and III:
- some are agonists of the CB2 receptor; in such a case, the radical R₁ represents preferably an alkyl having 1 to 6 carbon atoms, more preferably methyl or t-butyl. Preferably, said compounds of the formula (I) are chosen from the group consisting of: *N*3-(1-Adamantyl)-6-*tert*-butyl-1-pentyl-4-oxo-1,4-dihydropyridine-3-carboxamide **(Compound 23)**; *N*3-(Cyclohexyl)-6-*tert*-butyl-1-pentyl-4-oxo-1,4-dihydropyridine-3-carboxamide **(Compound 24)** and *N*3-(1-Adamantyl)-6-methyl-1-pentyl-4-oxo-1,4-dihydropyridine-3-carboxamide **(Compound 33).**
- some are inverse agonists of the CB2 receptor; in such a case, the radical R₁ represents preferably an aryl, more preferably a phenyl or a 4-Cl-phenyl.
Preferably, said compounds of the formula (I) are chosen from the group consisting of: (R,S)-*N*3-(1-(1-Adamantyl)ethyl)-4-oxo-1-pentyl-6-phenyl-1,4-dihydropyridine-3-carboxamide **(Compound 7)**, *N*3-((1-Adamantyl)methyl)-4-oxo-1-pentyl-6-phenyl-1,4-dihydropyridine-3-carboxamide **(Compound 9)**, *N*3-(1-(3,5-Dimethyl)adamantyl)-4-oxo-1-pentyl-6-phenyl-1,4-dihydropyridine-3-carboxamide **(Compound 10),** *N*3-(1-Adamantyl)-1-butyl-4-oxo-6-phenyl-1,4-dihydropyridine-3-carboxamide **(Compound 11)**, *N*3-(1-Adamantyl)-4-oxo-1-pentyl-6-phenyl-1,4-dihydropyridine-3-carboxamide **(Compound 12),** *N*3-(1-Adamantyl)-1-hexyl-4-oxo-6-phenyl-1,4-dihydropyridine-3-carboxamide **(Compound 13),** *N*3-(Cyclohexyl)-4-oxo-1-pentyl-6-phenyl-1,4-dihydropyridine-3-carboxamide **(Compound 14),** (R,S)-4-Oxo-1-pentyl-6-phenyl-*N*3-(1-(1,2,3,4-tetrahydronaphthyl))-1,4-dihydropyridine-3-carboxamide **(Compound 15)**, (R)-4-Oxo-1-pentyl-6-phenyl-*N*3-(1-(1,2,3,4-tetrahydronaphthyl))-1,4-dihydropyridine-3-carboxamide **(Compound 16)**, (S)-4-Oxo-1-pentyl-6-phenyl-*N*3-(1-(1,2,3,4-tetrahydronaphthyl))-1,4-dihydropyridine-3-carboxamide **(Compound 17)**, 4-Oxo-1-pentyl-6-phenyl-*N*3-(piperidin-1-yl)-1,4-dihydropyridine-3-carboxamide **(Compound 18),** 4-Oxo-1-pentyl-6-phenyl-*N*3-(3(trifluoromethyl)phenyl)-1,4-dihydropyridine-3-carboxamide **(Compound 19),** *N*3-(1-Adamantyl)-6-(4-chlorophenyl)-1-pentyl-4-oxo-1,4-dihydropyridine-3-carboxamide **(Compound 20),** 6-(4-Chlorophenyl)-*N*3-cyclohexyl-4-oxo-1-pentyl-1,4-dihydropyridine-3-carboxamide **(Compound 21),** 6-(4-Chlorophenyl)-4-oxo-1-pentyl-*N*3-(3(trifluoromethyl)phenyl)-1,4-dihydropyridine-3-carboxamide **(Compound 22),** *N*3-(1-Adamantyl)-1-pentyl-6-phenyl-4-thioxo-1,4-dihydropyridine-3-carboxamide **(Compound 25),** *N*3-(Cyclohexyl)-1-pentyl-6-phenyl-4-thioxo-1,4-dihydropyridine-3-carboxamide **(Compound 26),** *Tert*-butyl 4-oxo-1-pentyl-6-phenyl-1,4-dihydropyridin-3-yl carbamate **(Compound 27),** 5-Amino-1-pentyl-2-phenyl-1*H*-pyridin-4-one **(Compound 28)**, and *N*-(4-Oxo-1-pentyl-6-phenyl-1,4-dihydropyridin-3-yl)cyclohexanecarboxamide **(Compound 30)**
- for some of them listed in Table IV below, the Eₘₐₓ has not yet been measured; therefore the type of activity is not yet known; however they are considered to have an affinity for the CB2 receptor.

**Table IV. Compounds with not yet known activity (agonist or reverse agonist)**

| **Compound No.** | **X** | **Y** | **R₁** | **R₂** | **R₃** | **R₄** |
|---|---|---|---|---|---|---|
| **6** | O | 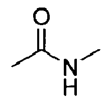 | 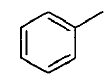 | H | C₂H₅ | 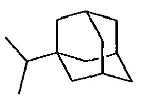 |
| **8** | O | 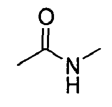 | 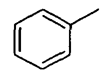 | H | 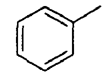 | 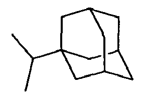 |
| **29** | O | 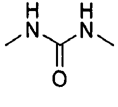 | 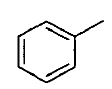 | H | C₅H₁₁ | 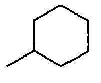 |
| **35** | O | 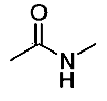 | CH₃ | I | C₅H₁₁ | 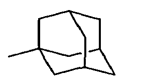 |
| **36** | O | 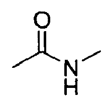 | CH₃ | C₄H₇ | C₅H₁₁ | 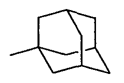 |
| **37** | O | 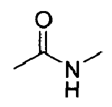 | CH₃ | 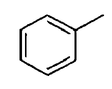 | C₅H₁₁ | 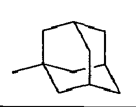 |
| **38** | O | 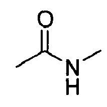 | CH₃ | *p*-tolyl | C₅H₁₁ | 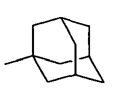 |
| **39** | O | 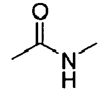 | CH₃ | cyanophényl | C₅H₁₁ | 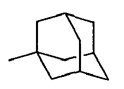 |

The Eₘₐₓ value is defined as the percentage of stimulation of [³⁵S]-GTPγS binding (basal value set at 100%). An agonist of the CB2 receptor presents an Eₘₐₓ value of more than 100 %, preferably around 135% and an inverse agonist of the CB2 receptor is a compound with an Eₘₐₓ value of less than 100 %, preferably around 65%. The Eₘₐₓ value is determined by a CB2 functional activity assay (19, 20).

Pharmaceutically acceptable salts include:
- those obtained by reacting the basic compounds of the formula (I), with an inorganic or organic acid to form a salt, for example, salts of hydrochloric acid, sulfuric acid, phosphoric acid, methane sulfonic acid, camphor sulfonic acid, oxalic acid, maleic acid, succinic acid, citric acid, formic acid, hydrobromic acid, benzoic acid, tartaric acid, fumaric acid, salicylic acid, mandelic acid, and carbonic acid; and
- those obtained by reacting acid compounds of the formula (I) with an appropriate base to form, e.g., sodium, potassium, calcium, magnesium, ammonium, and chlorine salts.

Those skilled in the art will further recognize that acid addition salts of the compounds of the formula (I) may be prepared by reaction of the compounds with an appropriate inorganic or organic acid via any of a number of known methods. Alternatively, alkali and alkaline earth metal salts can be prepared by reacting the compounds of the formula (I) with the appropriate base via a variety of known methods.

The following are further examples of acid salts that can be obtained by reaction with inorganic or organic acids: acetates, adipates, alginates, citrates, aspartates, benzoates, benzenesulfonates, bisulfates, butyrates, camphorates, digluconates, cyclopentanepropionates, dodecylsulfates, ethanesulfonates, glucoheptanoates, glycerophosphates, hemisulfates, heptanoates, hexanoates, fumarates, hydrobromides, hydroiodides, 2-hydroxy-ethanesulfonates, lactates, maleates, methanesulfonates, nicotinates, 2-naphtalenesulfonates, oxalates, palmoates, pectinates, persulfates, 3-phenylpropionates, picrates, pivalates, propionates, succinates, tartrates, thiocyanates, tosylates, mesylates and undecanoates.

The compounds of the formula (I) may exist as stereoisomers wherein asymmetric or chiral centers are present. These stereoisomers are "R" or "S" depending on the configuration of substituents around the chiral carbon atom. The terms "R" and "S" used herein are configurations as defined in IUPAC 1974 Recommendations for Section E, Fundamental Stereochemistry, Pure Appl Chem., 1976, 45: 13-30. The present invention contemplates various stereoisomers (including enantiomers and diastereoisomers) and mixtures thereof.

One of ordinary skill in the art will recognize that compounds of formula (I) can exist in different tautomeric and geometrical isomeric forms. All of these compounds, including *cis* isomers, *trans* isomers, diastereoisomeric mixtures, racemates, nonracemic mixtures of enantiomers, substantially pure, and pure enantiomers, are within the scope of the present invention. Substantially pure enantiomers contain no more than 5% w/w of the corresponding opposite enantiomer, preferably no more than 2% W/W, most preferably no more 1% W/W.

The optical isomers can be obtained by resolution of the racemic mixtures according to conventional processes, for example, by the formation of diastereoisomeric salts using an optically active acid or base or formation of covalent diastereoisomers. Examples of appropriate acids are tartric, diacyltartaric, dibenzoyltartaric, ditoluoyltartaric and camphosulfonic acid. Mixtures of diastereoisomers can be separated on the basis of their physical and/or chemical differences by methods known to those skilled in the art, for example, by chromatography or fractional crystallization. The optically active bases or acids are then liberated from the separated diastereoisomeric salts. A different process for separation of optical isomers involves the use of chiral chromatography (e.g. chiral HPLC columns), with or without conventional derivation, optimally chosen to maximize the separation of enantiomers. Suitable chiral HPLC columns are manufactured by Diacel, e.g., Chiracel OD and Chiracel OJ among many others, all routinely selectable. Enzymatic separations, with or without derivitization, are also useful. The optically active compounds of the formula (I) can likewise be obtained by utilizing optically active starting materials in chiral synthesis processes under reaction conditions which do not cause racemization.

Within the present invention it is to be understood that compounds of the formula (I) may exhibit the phenomenon of tautomerism. Thus, the invention encompasses any tautomeric or stereoisomeric form of compounds of the formula (I), as well as mixtures thereof.

One of ordinary skill in the art will also recognize that some of the compounds of formula (I) can exist in different polymorphic forms. As known in the art, polymorphism is an ability of a compound to crystallize as more than one distinct crystalline or "polymorphic" species. A polymorph, is a solid crystalline phase of a compound with at least two different arrangements or polymorphic forms of that compound molecule in the solid state. Polymorphic forms of any given compound are defined by the same chemical formula or composition and are as distinct in chemical structure as crystalline structures of two different chemical compounds.

One of ordinary skill art will further recognize that compounds of formula (I) can exist in different solvate forms. Solvates of the compounds of the invention may also form when solvent molecules are incorporated into the crystalline lattice structure of the compound molecule during the crystallization process.

The invention also relates to a process of preparation of compounds of the formula (I) wherein R₂ represents a hydrogen atom and Y represents CONH comprising the following steps:
(i) condensation of ethyl acetoacetate with N,N-dimethylformamide/dimethylsulfate adduct and triethylamine,
(ii) addition of the product of step (i) on the appropriate acyl chloride with subsequent cyclisation,
(iii) aminolysis of the product of step (ii),
(iv) facultatively thionation of the product of step (iii),
(v) saponification of the ethyl functions of the products of steps (iii) or (iv), and
(vi) amidation of the product of step (v).

Said process is illustrated in scheme 1 below: 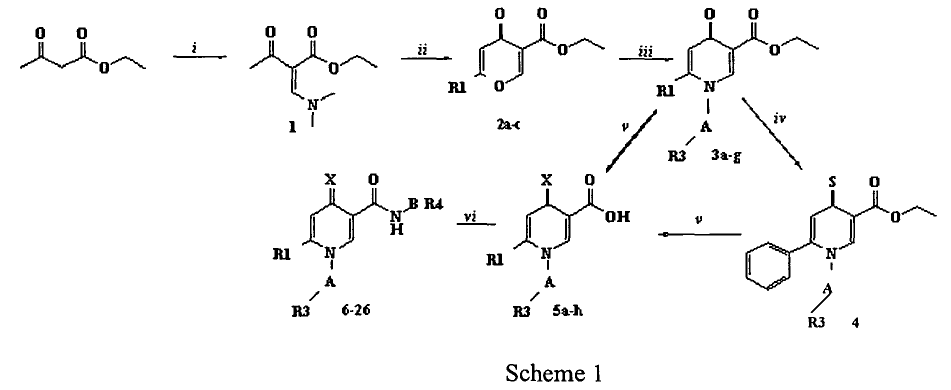 Enaminone **1** (product of step (i)) can be obtained by reaction of ethyl acetoacetate with the *N,N-*dimethylformamide/dimethylsulfate adduct (DMF/DMS) in combination with triethylamine.

Step (ii) consists of a subsequent deprotonation of **1,** preferably with a strong coordinating base lithium hexamethyldisilazide (LHMDS) in the presence of the appropriate acyl chloride preferably at -70°C followed by acidification preferably at room temperature and leads to **compounds 2a-c.**

Aminolysis in acidic conditions (step (iii)) leads to pyridine-4-ones **3a-g** in acceptable yields. The 6-phenyl-pyridine-4-thione analogue **4** was synthesized with very good yields from the corresponding pyridine-4-one **(3c)** by a thionation reaction using phosphorus pentasulfide as reagent (step (iv)).

After saponification of the ethyl ester functions of compounds **3a-g** and **4** (sodium hydroxide) (step (v)), the resulting carboxylic acids **5a-h** were engaged in an amidation reaction with the appropriate amines under peptidic conditions to obtain target amide compounds **6-26** (step (vi)).

The invention also relates to a process of preparation of compounds of the formula (I) wherein R₂ represents a hydrogen atom, Y represents NHCO or NHCONH, n=0 and R₃ represents an alkyl, for instance C₅H₁₁, said process comprising steps i) to (v) as specified above and further on the following steps according to scheme 2 below:
(vi) Curtius rearrangement of carboxylic acid into carbamate,
(vii) deprotection (Boc proctective group) of the product of step (i) into amine, and
(viii) coupling reaction of the product of step (ii) into urea or amide with a isocyanate or a carboxylic acid derivative.

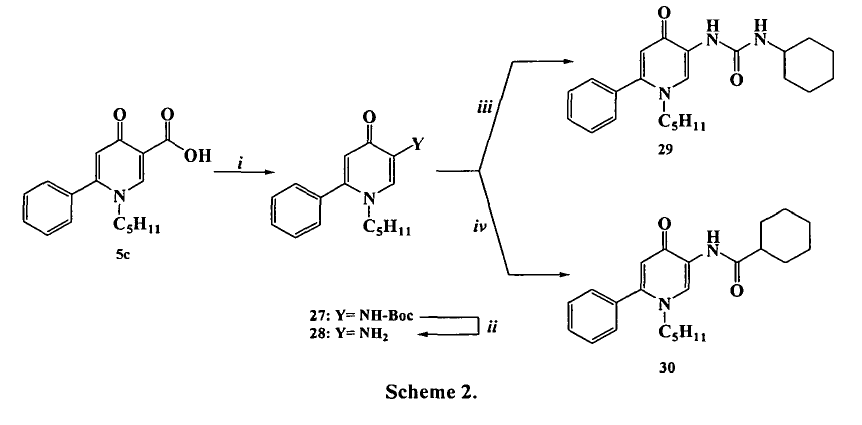

Carboxylic acid **5c** (Scheme 2) was converted to the carbamate **27** (Boc protected amine) *via* a Curtius rearrangement using diphenylphosphonyl azide and potassium *tert*-butoxide as reagents and *tert*-butanol as solvent. Treatment of **27** with hydrochloric acid in isopropanol followed by neutralization led to the amine **28.** The latter reacted either with cyclohexylisocyanate in alkaline medium to give urea **29** or with cyclohexyl carboxylic acid under peptidic conditions to afford the "reverse amide"**30.**

The invention also relates to a process of preparation of compounds of the formula (I) wherein R₁ represents an alkyl, for instance a methyl, R₂ represents a hydrogen atom, a iodine atom, a cyclopropyl, a phenyl, a para-tolyl or a meta-cyanophenyl, n=0, R₃ represents an alkyl, for instance C₅H₁₁, Y represents CONH, m=0 and R₄ represents an adamantyl. This process may apply for other R₃ and R₄ as defined above.

For instance, this process includes the following steps when R₂ represents a hydrogen atom:
(i) condensation of 4-hydroxy-6-methyl-2-pyrone with dimethylformamide dimethyl acetal (DMF-DMA),
(ii) aminolysis of the product of step (i),
(iii) coupling reaction of the product of step (ii) into amide with a carboxylic acid derivative.

This process includes the following steps when R₂ represents a iodine atom:
(i) condensation of 4-hydroxy-6-methyl-2-pyrone with dimethylformamide dimethyl acetal (DMF-DMA),
(ii) aminolysis of the product of step (i),
(iii) iodination of the product of step (ii) (step iv in scheme 3) and
(iv) coupling reaction of the product of step iii) into amide with a carboxylic acid derivative (step (iii) in scheme 3)

This process inludes the following steps when R₂ represents a cyclopropyl, a phenyl, a para-tolyl or a meta-cyanophenyl:
(i) condensation of 4-hydroxy-6-methyl-2-pyrone with dimethylformamide-dimethyl acetal (DMF-DMA),
(ii) aminolysis of the product of step (i),
(iii) iodination of the product of step (ii) (step iv in scheme 3),
(iv) coupling reaction of the product of step iii) into amide with a carboxylic acid derivative (step iii in scheme 3) and
(v) Suzuki cross-coupling reaction. 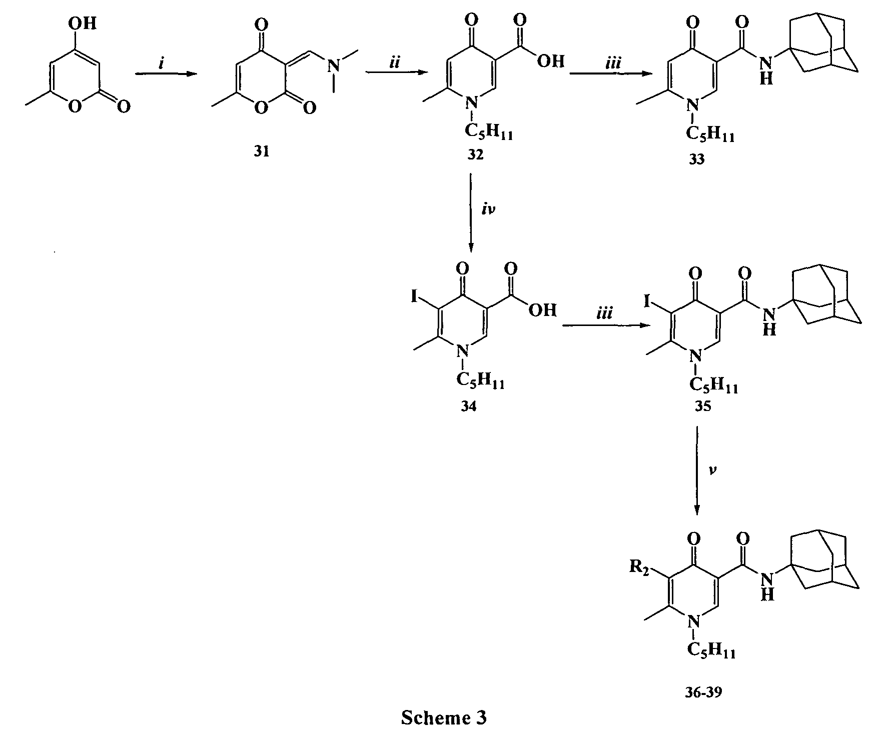
The synthesis of compounds **33, 35-39** was performed as outlined in Scheme 3 in three or five steps using a methodology adapted from the literature (17, 22-24).

The commercially available 4-hydroxy-6-methyl-2-pyrone was reacted with *N,N*-dimethylformamide dimethyl acetal in mild conditions to give **31,** which, when treated with n-pentylamine, under alkaline conditions followed by acidification (1N HCl) gave the carboxylic acid **32.** The latter reacted either with 1-aminoadamantane hydrochloride under peptidic conditions to give the target amide **33** or with iodine monochloride (compound **34**) and transformed to amide **35.** Finally, Suzuki cross-coupling reaction with appropriate boronic acids allowed the isolation of compounds **36-39** at high yields.

In order to use the compounds of the formula (I) in therapy, they are formulated into a pharmaceutical composition in accordance with standard pharmaceutical practice.
Thus, the present invention also provides a pharmaceutically composition, characterized in that it comprises at least a compound of formula (I) and at least a pharmaceutically acceptable vehicle.

A pharmaceutical composition of the invention, which may be prepared by a mixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusable solutions or suspensions or suppositories. Orally administrable compositions are generally preferred.

Tablets and capsules for oral administration may be in unit dose form, and may contain conventional excipients, such as binding agents, filters, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising a compound of the invention or pharmaceutically acceptable salt thereof and a vehicle. The compound, depending on the vehicle and concentration used, can be either suspended or dissolved in the vehicle. In preparating solutions, the compound can be dissolved for injection and filter sterilised before filling into a suitable vial or ampoule and sealing. Advantageously, adjuvants such as local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilate uniform distribution of the compound.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.05 to 200 mg, for example 20 to 40 mg; and such unit doses will preferably be administrated more than once a day may be required; and such therapy may extend for a number of weeks or months.

The invention also relates to compounds of the formula (I) as drugs.

The invention also relates to use of compounds of the fomula (I) for the preparation of a drug for treating diseases directly or indirectly associated with the modification (increase or decrease) of the activity of the CB2 receptor.

Thus the invention also relates to a compound of formula I for use in the treatment of conditions which are mediated by the activity of CB2 receptor.

The term "treatment" as used herein includes the treatment of established disorders and also includes the prophylaxis thereof.

The term "prophylaxis" is used herein to mean preventing symptoms in an already afflicted subject or preventing recurrence of symptoms in an afflicted subject and is not limited to complete prevention of an affliction.

The invention relates to the use of a compound of formula (I) or a pharmaceutically salt thereof, for the manufacture of a therapeutic agent for the treatment or prevention of a condition such as an immune disorder, an inflammatory disorder, pain, rheumatoid arthritis, multiple sclerosis, inflammatory bowel diseases, osteoarthritis or osteoporosis.

The invention also relates to the use of a compound of formula (I) for the preparation of a drug for treating or preventing the following diseases:
- immunological diseases such as autoimmune diseases, immunological deficiency diseases or organ transplantation;
- neurological diseases, such as brain edema, particularly tumor-related brain edema, multiple sclerosis, acute encephalomyelitis, meningitis, acute spinal cord injury, trauma, dementia, particularly degenerative dementia (including senile dementia, Alzheimer's disease, Parkinson's disease and Creutzfeldt-Jacob disease; Huntington's chorea, Pick's disease, motor neuron disease), vascular dementia (including multi-infarct dementia) as well as dementia associated with intracranial space occupying lesions; infections and related conditions (including HIV infection), Guillain-Barre syndrome, myasthenia gravis, and various forms of seizures, e.g., nodding spasms;
- amyotrophic lateral sclerosis (ALS) and neuroinflammation;
- neurodegeneration following stroke, cardiac arrest, pulmonary bypass, traumatic brain injury and spinal cord injury;
- tinnitus;
- kidney dysfunction (nephritis, particularly mesangial proliferative, glomerulonephritis, nephritic syndrome);
- liver dysfunction (hepatitis, cirrhosis);
- gastrointestinal disorders (diarrhoea) and colon cancer;
- pain and preferably chronic inflammatory pain (e.g. pain associated with rheumatoid arthritis, osteoarthritis, rheumatoid spondylitis, gouty arthritis and juvenile arthritis) including the property of disease modification and joint structure preservation, musculoskeletal pain, lower back and neck pain, sprains and strains, neuropathic pain, sympathetically maintained pain, myositis, pain associated with cancer and fibromyalie, pain associated with migraine, pain associated with influenza or other viral infections, such as the common cold, rheumatic fever, pain associated with functional bowel disorders such as non-ulcer dyspepsia, non-cardiac chest pain and irritable bowel syndrome (IBS), pain associated with myocardial ischemia, post operative pain, headache, toothache, and dysmenorrhea;
- inflammation, for example in skin conditions (e.g. sunburn, bums, eczema, dermatitis, psoriasis); ophthalmic diseases such as glaucoma, retinitis, retinopathies, uveitis and of acute injury to the eye tissue (e.g. conjunctivitis); lung disorders (e.g. asthma, bronchitis, emphysema, allergic rhinitis, respiratory distress syndrome, pigeon fancier's disease, farmer's lung, chronic obstructive pulmonary disease (COPD), gastrointestinal tract disorders (e.g. aphthous ulcer, Crohn's disease, atopic gastritis, gastritis varialoforme, ulcerative colitis, coelliac disease, regional ileitis, irritable bowel syndrome, inflammatory bowel disease(IBD)), migraine, perirteritis nodosa, thyroiditis, aplastic anemia, Hodgkin's disease, sclerodoma, myaesthenia gravis, multiple sclerosis, sorcoidosis, nephrotic syndrome, Bechet's syndrome, polymyositis, gingivitis, myocardial ischemia, pyrexia, systemic lupus erythematosus, tendonitis, bursitis and Sjogren's syndrome;
- tumors (e.g. tumor of immune origin), more specifically in the treatment of cancers of immune origin (e.g. malignant lymphoblastic disease);
- bladder hyperrelexia following bladder inflammation; and
- psychiatric diseases for example schizophrenia, depression (which term is used herein to include bipolar depression, unipolar depression, single or recurrent major depressive episodes with or without psychotic features, catatonic features, melancholic features, atypical features, or postpartum onset, seasonal affective disorder, dysthymic disorders with early or late onset and with or without atypical features, neurotic depression and social phobia, depression accompanying dementia for example of the Alzheimer's type, schizoaffective disorder or the depressed type, and depressive disorders resulting from general medical conditions including, but not limited to, myocardial infarction, diabetes, miscarriage or abortion etc...), anxiety disorders (including generalised anxiety disorder and social anxiety disorder), panic disorder, agoraphobia, social phobia, obsessive compulsive disorder and post-traumatic stress disorder, memory disorders, including dementia, amnesic disorders and age-associated memory impairment, disorders of eating behaviours (including anorexia, nervosa and bulimia nervosa, sexual dysfunction, sleep disorders (including disturbances of circadian rhythm, dyssomnia, insomnia, sleep apnea and narcolepsy), withdrawal from abuse of drugs such as cocaine, ethanol, nicotine, benzodiazepines, alcohol, caffeine, phencyclidine (phencyclidine-like compounds), opiates (e.g. cannabis, heroin, morphine), amphetamine, or amphetamine-related drugs (e.g. dextroamphetamine, methylamphetamine) or a combination thereof.

The compounds of formula (I) may also be effective in increasing the latency of HIV infection.

Compounds of formula (I) may also be useful in the treatment of fever.

Compounds of formula (I) may also be useful in the treatment of diseases of abnormal platelet function (e.g. occlusive vascular diseases).

Compounds of formula (I) may also be useful in the treatment of neuritis, heart burn, dysphagia, pelvic hypersensitivity, urinary incontinence cystitis or pruritis.

Compounds of formula (I) may also be useful in the treatment of a drug with diuretic action.

Compounds of formula (I) may also be useful in the treatment of impotence or erectile dysfunction.

Compounds of formula (I) may also be useful for attenuating the hemodynamic side effects of non-steroidal anti-inflammatory drugs (NSAID's) and cyclooxygenase-2 (COX-2) inhibitors.

Compounds of formula (I) may be useful as analgesics.

Compounds of the invention which bind to the CB2 receptor may also have disease modification or joint structure preservation properties in multiple sclerosis, rheumatoid arthritis, osteoarthritis, rheumatoid spondylitis, gouty arthritis and juvanil arthritis.

Compounds of the invention may be particularly useful in the treatment of neuropathic pain. Neuropathic pain syndromes can develop following neuronal injury and the resulting pain may persist for months or years, even after the original injury has healed. Neuropathic pain syndromes are traditionally classified according to the disease or event that precipitated them. Neuropathic pain syndromes include: diabetic neuropathy, sciatica, non-specific lower back pain, multiple sclerosis pain, fibromyalgia, HIV-related neuropathy, post-herpethic neuralgia, trigeminal neuralgia, and pain resulting from physical trauma, amputation, cancer, toxins or chronic inflammatory conditions. These conditions are difficult to treat and although several drugs are known to have limited efficacy, complete pain control is rarely achieved. The symptoms of neuropathic pain are incredibly heterogeneous and are often describe as spontaneous shooting and lancinating pain, or ongoing, burning pain.. In addition, there is pain associated with normally non-painful sensations such as "pins and needles" (paraesthesias and dysesthesias), increased sensitivity to touch (hyperesthesia), painful sensation following innocuous stimulation (dynamic, static or thermal allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

According to a further aspect of the invention, it relates to a method of treating a mammal, for example a human suffering from a immune disorder, an inflammatory disorder, pain, rheumatoid arthritis, multiple sclerosis, osteoarthritis or osteoporosis, inflammatory bowel diseases, which method comprises administrating to said subject an effective amount of a compound of formula (I) or a pharmaceutically acceptable salt thereof.

In one embodiment the pain is selected from inflammatory pain, visceral pain (e.g. Irritable Bowel Syndrome), cancer pain, neuropathic pain, lower back pain, muscular skeletal, post operative pain, acute pain and migraine. For example, the inflammatory pain is pain associated with rheumatoid arthritis, osteoarthritis or inflammatory bowel diseases.

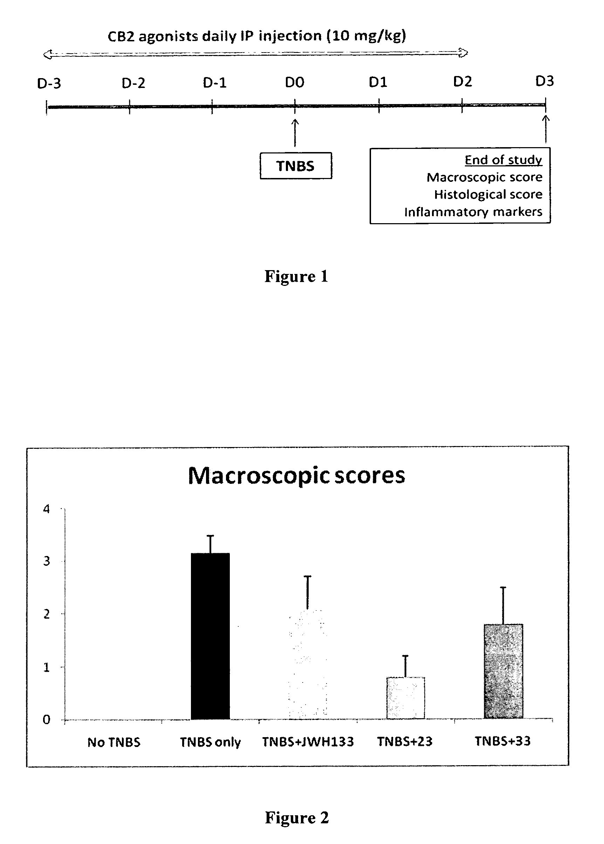

The invention will be better understood when regarding the following non-limitative examples in relation with figures 1 to 6:
- Figure 1 shows a study outline of the CB2 agonists tested in TNBS-induced colitis of example 4.
- Figure 2 shows the macroscopic scores: Mice injected with TNBS showed macroscopic colitis reflected by thickening of the bowel and ulceration areas. The positive control JWH-133 induced a 34% decrease in colitis macroscopic scores (3,1 ± 0,3 vs 2,1 ± 0,6, p≤0,05). The new derivatives (compounds **23** and **33**) induced respectively a 74 and 42% decrease in colitis macroscopic scores (3,1 ± 0,3 vs respectively 0,8 ± 0,4, p≤0,01 and 1,8 ± 0,7, p≤0,01).

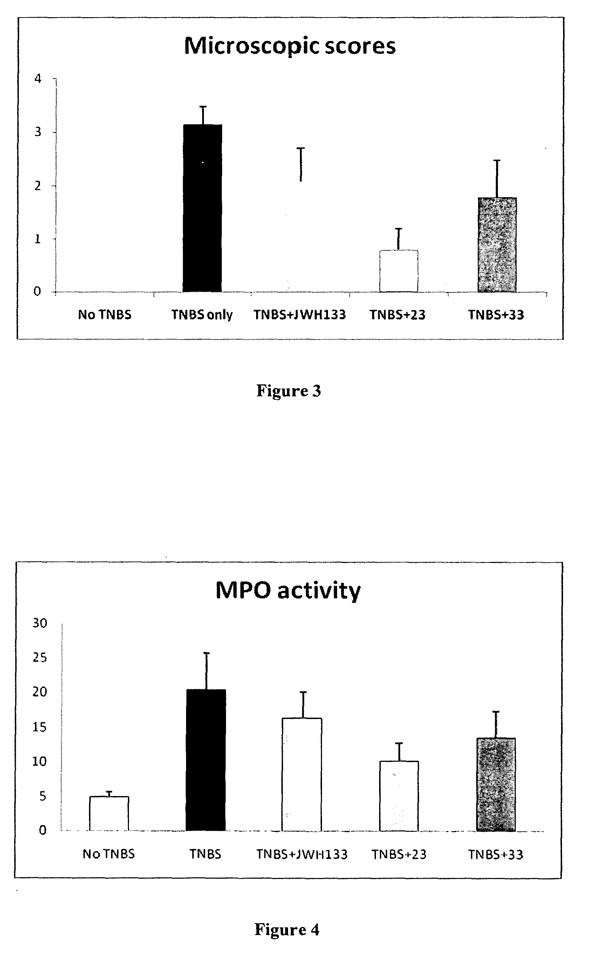

- Figure 3 shows histological scores of compounds **23** and **33** as well as the positive control JWH133. Compounds **23** and **33** as well as the positive control JWH133 induced a decrease of histological scores as compared to TNBS only (3,7 ± 1 vs 1,8 ± 0,6, p≤0,05 for compound **23** and 2,5 ± 0,8, p≤0,01 for compound **33**). Noteworthy is the significant 51% inhibition of colitis microscopic score by compound **23.**
- Figure 4 shows the MPO activity: The MPO activity reflecting neutrophils content is significantly 50% and 34% decreased by compounds **23** and **33** treatment respectively (20 ± 5 for TNBS only vs 10 ± 3, p≤0,05 for compounds **23** and 14 ± 4 for compound **33**).

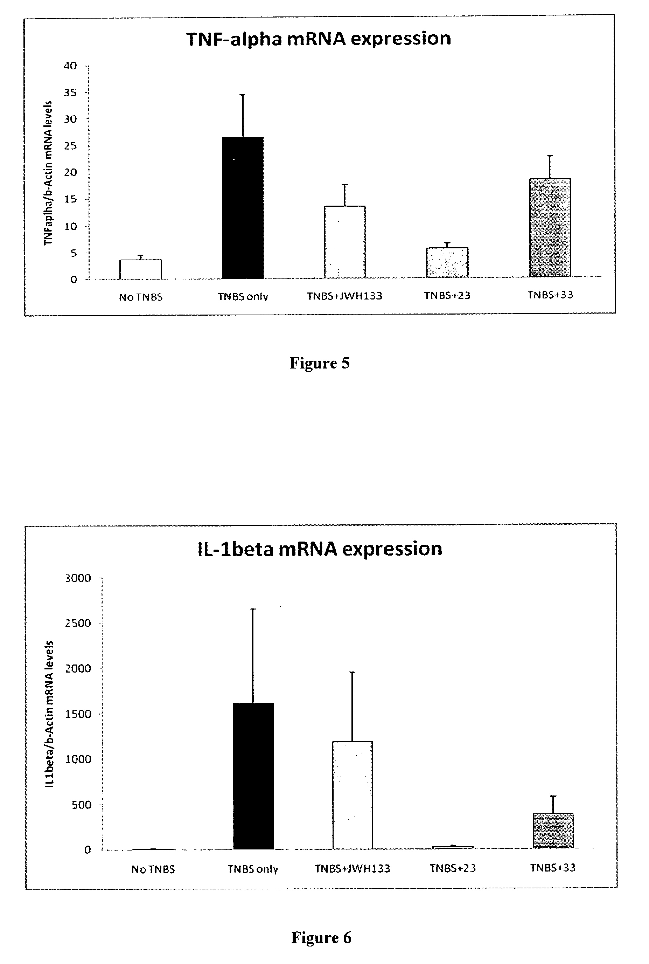

- Figures 5 and 6 show TNF-alpha and IL-1beta mRNA expression as inflammatory marker: Treatment with compound **23** significantly decreased by 79 and 99% the levels of pro-inflammatory cytokines TNF-alpha and IL-1 beta in colon (TNF-alpha: 26 ± 8 for TNBS only vs 6 ± 1, p≤0,01 for compound **23** and 18 ± 4 for compound **33;** IL-1beta: 1610 ± 1041 for TNBS only vs 22 ± 11, p≤0,01 for compound 23 and 385 ± 192 for compound **33).**

### Example 1: Synthesis of compounds 6-26

The pyridin-4-one scaffold was prepared from ethyl acetoacetate as previously described (21). The synthesis of the 6-phenyl, 6-(4-chlorophenyl) or *6-tert-*butyl substituted 4-oxo-1,4-dihydropyridine and 4-thioxo-1,4-dihydropyridine **6-26** is emphasized in Scheme 1 above.

**Reagents & Conditions of Scheme 1.** (*i*) DMF/DMS, Et₃N, CH₂Cl₂, rt, 2 h, 79%; (*ii*) a- LHMDS, R₁COCl, THF, -78 °C, 15 min, b- 3N HCl, Et₂O, rt, 45 min, 52-67%; (*iii*) R₃-NH₂, AcOH/EtOH 1:3 reflux, 1 h, 57-77%; (iv) (when compound 3c) P₄S₁₀, pyridine, reflux, 12 h, 90%; (v) 10% NaOH /EtOH v/v, reflux, 6 h, 77-96%; (*vi*) a- HBTU(2-(1H-benzotriazol-1-yl)-1,1,3,3-tetramethyluronium hexafluorophosphate), HOBt(hydroxybenzotriazole), DIPEA(diisopropylethylamine), DMF, rt, 4-6 h, b- R₄-NH₂, rt, 12 h, 28-74 %.

**Compound 1** was prepared from ethyl acetoacetate as previously described by McCombie et al., (21) by reaction of ethyl acetoacetate with the *N,N-*dimethylformamide/dimethylsulfate adduct (DMF/DMS) in combination with triethylamine (step i).

Subsequent deprotonation of compound 1 with a strong coordinating base (LHMDS) in the presence of the appropriate acyl chloride at -70°C followed by acidification at room temperature led directly to **compounds 2a-c** (step ii). Aminolysis in acidic conditions afforded **compounds 3a-g** in acceptable yields. **Compound 4** was synthesized from the **compound 3c** by a thionation reaction using phosphorus pentasulfide as reagent (step iv). After saponification of the ethyl ester functions of **compounds 3a-g** and 4 (step v), the resulting **compounds 5a-h** were engaged in an amidation reaction with the appropriate amines under peptidic conditions to afford **compounds 6-26** (step vi). More precisely, to a solution of **compounds 5a-h,** in dry DMF, were added DIPEA (3 equiv.) and the coupling agents HOBt (0.5 equiv.), HBTU (1.5 equiv.). The resulting mixture was stirred at room temperature until TLC (95/5 CH₂Cl₂/MeOH) showed the starting material to be consumed (c.a 3 h). The amine was then added and the solution was stirred at room temperature for 12 h. The solvent was removed under reduced pressure and the residue taken up in water and extracted with CH₂Cl₂. The organic phase was washed, with saturated aqueous NaHCO₃ solution, with 1N aqueous HCl and water. The organic extract was dried over MgSO₄ and concentrated in vacuo to a brown oil. The crude material was purified by TLC using the appropriate eluent (dichloromethane/methanol 9:1) and recristallized in heptane to obtain **compounds 6-26.**

### Example 2: Synthesis of compounds 27-30

**Compounds 27, 28, 29 and 30** were obtained following the protocol shown in scheme 2 above. **Compounds 5a-h** were converted to **compound 27** via a Curtius rearrangement using diphenylphosphonyl azide and potassium *tert*-butoxide as reagents and *tert*-butanol as solvent. Treatment of **compound 27** with hydrochloric acid in isopropanol followed by neutralization led to the compound 28. The latter reacted either with cyclohexylisocyanate in alkaline medium to give **compound 29** or with cyclohexyl carboxylic acid under peptidic conditions to obtain the "reverse amide"**30.**

**Reagents & Conditions of scheme 2.** (*i*) DPPA(diphenylphosphorylazide), *t*-BuOK, *t*-BuOH, reflux, 12 h, 38%; (*ii*) a- 5N HCl, isopropanol, rt, 14 h, b- 10% NaOH, 95%; (*iii*) cyclohexyl isocyanate, Et₃N, CHCl₃, rt, 48 h, 48%; (*iv*) a- cyclohexyl carboxylic acid, HBTU, HOBt, DIPEA, DMF, rt, 4 h, b- 28, rt, 12 h, 38%.

More precisely:
***Tert*-butyl 4-oxo-1-pentyl-6-phenyl-1,4-dihydropyridin-3-yl carbamate (compound 27):** To a solution of carboxylic acid **5c** (0.4 g, 1.4 mmol) in *tert*-butyl alcohol (20 mL) were added under nitrogen atmosphere potassium *tert-*butoxide (0.17 g, 1.7 mmol) and diphenylphosphoryl azide (0.36 mL, 1.7 mmol). The mixture was refluxed for 12 h, cooled to room temperature and diluted with EtOAc. The organic phase was washed with saturated aqueous NaHCO₃ solution and brine, dried over MgSO₄ and concentrated under reduced pressure to a yellowish oil. The latter was purified by silica gel chromatography (dichloromethane/methanol 95:5) to afford pure 27 (0.17 g, 38%) as a yellow oil.
**5-Amino-1-pentyl-2-phenyl-1*H*-pyridin-4-one (compound 28):**
   To a solution of 5N hydrochloric acid in isopropanol (20 mL) was added the carbamate compound 27 (0.1 g, 0.28 mmol). The mixture was stirred at room temperature for 14 h and then concentrated under reduce pressure. The resulting solid was solubilized in water and washed with Et₂O. The aqueous phase was alkalinized with 10% NaOH and extracted with EtOAc. The organic phase was dried over MgSO₄ and the solvent removed under reduced pressure to afford pure compound **28** as a white solid (0.068 g, 95%).
**1-Cyclohexyl-3-(4-oxo-1-pentyl-6-phenyl-1,4-dihydropyridin-3-yl)urea (compound 29):** To a 20 mL CHCl₃ solution of compound **28** (0.1 g, 0.4 mmol) and Et₃N (0.14 mL, 1 mmol) was added cyclohexyl isocyanate (0.1 mL, 0.8 mmol). The resulting mixture was stirred at room temperature for 48 h. The solvent was removed in vacuo, dichloromethane was added and the organic phase was washed with 1N hydrochloric acid solution, dried over MgSO₄ and the solvent removed under reduced pressure. The residue was purified by silica gel chromatography (ethyl acetate/cyclohexane 1:1) to give a white solid (0.075 g, 48%).
***N*-(4-Oxo-1-pentyl-6-phenyl-1,4-dihydropyridin-3-yl)cyclohexanecarboxamide (compound 30):** Compound **30** was obtained using the same procedure described for amides compounds **6-26.** Purification by silica gel chromatography (ethyl acetate/cyclohexane 1:1) is performed.

### Example 3: Synthesis of compounds 33 and 35-39

The synthesis of compounds **33, 35-39** was performed as outlined in Scheme 3 in three or five steps using a methodology adapted from the literature (17, 22-24).

**Reagents & Conditions of scheme 3.** (*i*) DMF-DMA, dioxane, rt, 4 h, 63%; (*ii*) *t*-BuOK, n-pentylamine, EtOH, reflux, 12 h, 73%; (*iii*) a- HBTU, HOBt, DIPEA, DMF, rt, 4 h, b- 1-aminoadamantane hydrochloride, rt, 12 h, 57-69%, (iv) ICl, CaCO₃, DMF, 50 °C, 12 h, 91%; R₂-B(OH)₂, K₃PO₄, Pcy₃, Pd(AcO)₂, Toluene/H₂O, 12 h, reflux, 74-92%.

The commercially available 4-hydroxy-6-methyl-2-pyrone was reacted with *N,N*-dimethylformamide dimethyl acetal in mild conditions to give **31,** which, when treated with n-pentylamine, under alkaline conditions followed by acidification (1N HCl) gave the carboxylic acid **32.** The latter reacted either with 1-aminoadamantane hydrochloride under peptidic conditions to give the target amide **33** or with iodine monochloride (compound **34**) and transformed to amide **35.** Finally, Suzuki cross-coupling reaction with appropriate boronic acids allowed the isolation of compounds **36-39** at high yields.

**General procedure for the preparation of carboxamides 33 and 35.** Compound **33** and **35** were obtained using the same procedure described for amides compounds **6-26.** In this case, carboxylic acids **32** and **34** were used. Purification by silica gel chromatography (dichloromethane/methanol 9:1).

### General procedure for the preparation of compounds 36-39.

Compound **35** (1 equiv), appropriate boronic acid (1.5 equiv), potassium phosphate (4 equiv), palladium acetate (0.25 equiv) and tricyclohexylphosphine (0.5 equiv) were suspended in a mixture of toluene/H₂0 (3:2). The mixture was refluxed for 12 h and the palladium acetate filtered. The resulting filtrate was then concentrated to dryness and the residue partitioned in H₂O-CHCl₃. The organic phase was washed both with water and brine, dried and evaporated. The crude material was chromatographied on silica gel (dichloromethane/methanol 95:5) to afford pure product.

### Example 4: synthesis of compounds 2-5, 31, 32 and 34 and characteristics

All commercial reagents and solvents were used without further purification. Analytical thin layer chromatography was performed on precoated Kieselgel 60F₂₅₄ plates (Merck) The spots were located by UV (254 and 366 nm) and/or with iodine. Silica gel 60 230-400 mesh purchased from Merck was used for column chromatography. Alumina silica gel (neutral) 150 mesh purchased from Sigma Aldrich was used for column chromatography in the case of the purification of compound **2c.** Preparative thick-layer chromatography (TLC) was performed using silica gel from Merck, the compounds were extracted from silica gel by the following solvent system: CHCl₃/MeOH 8:2. All melting points were determined with a Büchi 535 capillary apparatus and remain uncorrected. ¹H NMR spectra were obtained using a Brücker 300 MHz spectrometer, chemical shift (δ) were expressed in ppm relative to tetramethylsilane used as an internal standard, *J* values are in hertz, and the splitting patterns were designated as follows: s singlet, d doublet, t triplet, m multiplet. APCI⁺ (Atmospheric Pressure Chemical Ionization) mass spectra were obtained on an LC-MS system Thermo Electron Surveyor MSQ.

The ethyl 2-[(dimethylamino)methylene]-3-oxobutanoate **1,** pyran-4-ones **2a** (ethyl 4-oxo-6-phenyl-4*H*-pyran-3-carboxylate) and **2b** (ethyl 6-*tert*-butyl-4-oxo-4*H*-pyran-3-carboxylate) and pyridin-4-one **3e** (ethyl 4-oxo-1,6-diphenyl-1,4-dihydropyridine-3-carboxylate) were obtained using the same procedures previously described (21) with minor modifications. In the insant case, pyran-4-ones **2a-c** were too unstable on silica gel and could not be purified even by flash chromatography. It was found that pyran-4-ones **2a** and **2b** crystallize in diethyl ether at low temperature under reduced pressure.

**3-(Dimethylaminomethylene)-6-methyl-4-oxo-2-pyrone 31** was prepared according to a procedure already described. (17). Compound **31** crystallizes in a mixture of toluene/cyclohexane (2:8).

**Ethyl 6-(4-chlorophenyl)-4-oxo-4*H*-pyran-3-carboxylate 2c:** this compound was obtained using the same methodology as already described. (21)_{;} it was purified by neutral silica alumina gel (dichloromethane/ethyl acetate 1:1); beige powder (52%); mp 155°C; ¹H NMR (CDCl₃) δ 8.58 (s, 1H), 7.7 (d, 2H, *J* = 9.1 Hz), 7.5 (d, 2H, *J* = 9.1 Hz), 6.84 (s, 1H), 4.40 (q, 2H, *J* = 7.0 Hz), 1.41 (t, 3H, *J* = 7.0 Hz). LC-MS (APCI⁺) *m*/*z* 279.4 (MH⁺).

**General procedure for the preparation of ethyl 1-alkyl-6-aryl-4-oxo-1,4-dihydropyridine-3-carboxylate (3a-g):** to a solution of pyrone in EtOH/AcOH (3:1) was added, slowly, the appropriate alkylamine or aniline (2 equiv.) at 5°C. The mixture was refluxed for 1 h. After cooling to room temperature, solvents were evaporated and the residue partitioned in H₂O-CHCl₃. The organic phase was washed both with water and brine, dried and evaporated to leave an oil. The latter was chromatographied on silica gel to afford pure product.

More precisely:
**Ethyl 1-ethyl-4-oxo-6-phenyl-1,4-dihydropyridine-3-carboxylate (compound 3a):** Purification by silica gel chromatography (dichloromethane/ethyl acetate 1:1); beige solid (77%); mp 159°C; ¹H NMR (CDCl₃) δ 8.41 (s, 1H), 7.50 (m, 5H), 6.07 (s, 1H), 4.22 (q, 2H, *J* = 6.9 Hz), 3.81 (q, 2H, *J* = 7.4 Hz), 1.26 (t, 3H, *J* = 6.9 Hz), 1.04 (t, 3H, *J* = 7.4 Hz); LC-MS (APCI⁺) *m*/*z* 272.1 (MH⁺).
**Ethyl 1-butyl-4-oxo-6-phenyl-1,4-dihydropyridine-3-carboxylate (compound 3b):** Purification by silica gel chromatography (dichloromethane/ethyl acetate 1:1); brown oil (74%); ¹H NMR (CDCl₃) δ 8.22 (s, 1H), 7.48 (m, 3H) 7.32 (m, 2H), 6.42 (s, 1H), 4.39 (q, 2H, *J* = 7.2 Hz ), 3.74 (t, 2H, *J* = 7.6 Hz ), 1.52 (m, 2H), 1.38 (t, 3H, *J* = 7.2 Hz), 1.12 (m, 2H), 0.75 (t, 3H, *J* = 7.4 Hz). LC-MS (APCI⁺) *m*/*z* 300.2 (MH⁺).
**Ethyl 4-oxo-1-pentyl-6-phenyl-1,4-dihydropyridine-3-carboxylate (compound 3c):** Purification by silica gel chromatography (dichloromethane/ethyl acetate 1:1); brown oil (71%); ¹H NMR (DMSO-*d₆*) δ 8.40 (s, 1H), 7.51 (m, 5H), 6.08 (s, 1H), 4.22 (q, 2H, *J* = 7.0 Hz ), 3.81 (t, 2H, *J* = 7.6 Hz), 1.38 (m, 2H), 1.26 (t, 3H, *J* = 7.0 Hz), 1.02 (m, 4H), 0.69 (t, 3H, *J* = 7.0 Hz). LC-MS (APCI⁺) *m*/*z* 314.2 (MH⁺).
**Ethyl 4-oxo-1-hexyl-6-phenyl-1,4-dihydropyridine-3-carboxylate (compound 3d):** Purification by silica gel chromatography (dichloromethane/ethyl acetate 1:1); brown oil (68%); ¹H NMR (CDCl₃) δ 8.22 (s, 1H), 7.41 (m, 5H), 6.08 (s, 1H), 4.19 (q, 2H, *J* = 6.9 Hz), 3.93 (t, 2H, *J* = 7.3 Hz), 1.26-1.02 (m, 11H), 0.73 (t, 3H, *J* = 7.1 Hz). LC-MS (APCI⁺) *m*/*z* 328.2 (MH⁺).
**Ethyl 6-(4-chlorophenyl)-4-oxo-1-pentyl-1,4-dihydropyridine-3-carboxylate (compound 3f):** Purification by silica gel chromatography (dichloromethane/methanol 9:1); yellow oil (75%); ¹H NMR (CDCl₃) δ 8.22 (s, 1H), 7.50 (d, 2H, *J* = 8.8 Hz), 7.30 (d, 2H, *J* = 8.8 Hz), 6.40 (s, 1H), 4.40 (q, 2H, *J* = 7.1 Hz), 3.72 (t, 2H, *J* = 7.7 Hz), 1.55 (m, 2H), 1.40 (t, 3H, *J* = 7.1 Hz), 1.18 (m, 4H), 0.82 (t, 3H, *J* = 7.0 Hz). LC-MS (APCI⁺) *m*/*z* 348.2 (MH⁺).
**Ethyl 6-*tert*-butyl-4-oxo-1-pentyl-1,4-dihydropyridine-3-carboxylate (compound 3g):** A solution of pyrone ester (compound 2b) (3 g, 13.4 mmol), n-pentylamine (3.1 mL, 26.7 mmol) in EtOH (60 mL) and AcOH (40 mL) was refluxed for 4 h. The mixture was cooled to room temperature and the solvents were distilled off to leave a brown oil. Water was added and the product was extracted with CH₂Cl₂. The crude oil was chromatographied on silica gel (dichloromethane/methanol 98:2) to give a rust colored oil (2.24 g, 57%); ¹H NMR (CDCl₃) δ 8.12 (s, 1H), 6.55 (s, 1H), 4.38 (q, 2H, *J* = 7.1 Hz), 4.02 (t, 2H, *J* = 8.3 Hz), 1.81 (m, 2H), 1.40-1.34 (m, 16H), 0.94 (t, 3H, *J* = 6.7 Hz); LC-MS (APCI⁺) *m*/*z* 294.4 (MH⁺).
**Ethyl 1-pentyl-6-phenyl-4-thioxo-1,4-dihydropyridine-3-carboxylate (compound 4):** A mixture of compound 3c (2.6 g, 8.3 mmol) and phosphorus pentasulfide (3.7 g, 16.6 mmol) was reflux for 12 h in pyridine (80 mL). After cooling to room temperature, the solvent was removed under reduced pressure and the residue was partitioned in H₂O-EtOAc. The organic layer was washed with brine, dried and concentrated under reduced pressure to leave a rust colored oil. The latter was purified by silica gel chromatography (cyclohexane/ethyl acetate 9:1) to give an orange oil (2.46 g, 90%). ¹H NMR (DMSO-d₆) δ 8.22 (s, 1H), 7.54 (m, 5H), 7.06 (s, 1H), 4.26 (q, 2H, *J* = 7.0 Hz), 3.85 (t, 2H, *J* = 7.6 Hz), 1.45 (m, 2H), 1.28 (t, 3H, *J* = 7.0 Hz), 1.03-0.98 (m, 4H), 0.70 (t, 3H, *J* = 6.7 Hz). LC-MS (APCI⁺) *m*/*z* 330.8 (MH⁺).

**General procedure for the preparation of carboxylic acids (5a-h).** Esters **3a-g** and **4** were dissolved in ethanol and 10% NaOH (v/v). The mixture was refluxed for 6 h. After cooling to room temperature, EtOH was removed under reduced pressure and the residue was dissolved in water and washed with EtOAc. The aqueous phase was acidified (1N HCl pH 2) and extracted with EtOAc. The combined organic extracts were washed with water and brine, dried over MgSO₄ and concentrated under reduced pressure to afford essentially pure carboxylic acids **5a-h.**

**1-Ethyl-4-oxo-6-phenyl-1,4-dihydropyridine-3-carboxylic acid (5a).** Beige powder (96%); mp 108°C; ¹H NMR (CDCl₃) δ 12.56 (s, 1H), 8.51 (s, 1H) ; 7.54 (m, 5H); 6.09 (s, 1H); 4.02 (q, 2H, *J* = 7.4 Hz), 1.17 (t, 3H, *J* = 7.4 Hz). LC-MS (APCI⁺) *m*/*z* 244.2 (MH⁺).

**1-Butyl-4-oxo-6-phenyl-1,4-dihydropyridine-3-carboxylic acid (5b).** White powder (92%); mp 121°C; ¹H NMR (DMSO-d₆) δ 16.35 (s, 1H), 8.87 (s, 1H), 7.56 (m, 5H), 6.63 (s, 1H), 4.03 (t, 2H, *J* = 7.6 Hz), 1.43 (m, 2H),1.03 (m, 2H), 0.63 (t, 3H, *J* = 7.4 Hz). LC-MS (APCI⁺) *m*/*z* 272.1 (MH⁺).

**4-Oxo-1-pentyl-6-phenyl-1,4-dihydropyridine-3-carboxylic acid (5c).** Purification by silica gel chromatography (dichloromethane/ethyl acetate 1:1); white powder (88%); mp 115°C; ¹H NMR (CDCl₃) δ 13.5 (s, 1H), 8.4 (s, 1H), 7.48 (m, 5H), 6.08 (s, 1H), 3.81 (t, 2H, *J* = 7.1 Hz), 1.38 (m, 2H), 1.02-0.97 (m, 4H), 0.69 (t, 3H, *J* = 7.0 Hz). LC-MS (APCI⁺) *m*/*z* 286.1 (MH⁺).

**1-Hexyl-4-oxo-6-phenyl-1,4-dihydropyridine-3-carboxylic acid (5d).** Purification by silica gel chromatography (dichloromethane/ethyl acetate 1:1); beige oil (86%); ¹H NMR (DMSO-d₆) δ 16.35 (s, 1H), 8.88 (s, 1H), 7.56 (m, 5H), 6.63 (s, 1H), 4.02 (t, 2H, *J* = 7.6 Hz), 1.43-0.85 (m, 8H), 0.73 (t, 3H, *J* = 7.2 Hz). LC-MS (APCI⁺) *m*/*z* 300.1 (MH⁺).

**4-Oxo-1,6-diphenyl-1,4-dihydropyridine-3-carboxylic acid (5e).** White powder (94%); mp 182°C; ¹H NMR (CDCl₃) δ 13.20 (s, 1H), 8.76 (s, 1H), 7.42-7.21 (m, 10H), 6.82 (s, 1H). LC-MS (APCI⁺) *m*/*z* 292.3 (MH⁺).

**6-(4-Chlorophenyl)-4-oxo-1-pentyl-1,4-dihydropyridine-3-carboxylic acid (5f).** White powder (86%); mp 184°C; ¹H NMR (CDCl₃) δ 13.88 (s, 1H), 8.58 (s, 1H), 7.7 (d, 2H, *J* = 9.1 Hz), 7.5 (d, 2H, *J* = 9.1 Hz), 6.84 (s, 1H), 4.01 (t, 2H, *J* = 7.0 Hz ), 1.52 (m, 2H), 1.26 (m, 4H), 1.02 (t, 3H, 6.9 Hz). LC-MS (APCI⁺) *m*/*z* 320.5 (MH⁺).

**6-*tert*-Butyl-4-oxo-1-pentyl-1,4-dihydropyridine-3-carboxylic acid (5g).** Beige powder (77%); mp 89°C; ¹H NMR (DMSO-*d₆*) δ 11.97 (s, 1H), 8.66 (s, 1H), 6.68 (s, 1H), 4.31 (t, 2H, *J* = 8.2 Hz), 1.73 (m, 2H), 1.41 (s, 9H), 1.32 (m, 4H), 0.87 (t, 3H, *J* = 6.7 Hz). LC-MS (APCI⁺) *m*/*z* 266.3 (MH⁺).

**1-Pentyl-6-phenyl-4-thioxo-1,4-dihydropyridine-3-carboxylic acid (5h).** Purification by silica gel chromatography (dichloromethane/ethyl acetate 1:1); orange powder (84%); mp 108°C; ¹H NMR (DMSO-*d₆*) δ 13.75 (s, 1H), 9.00 (s, 1H), 7.59 (m, 5H), 7.40 (s, 1H), 4.10 (t, 2H, *J* = 8.4 Hz), 1.49 (m, 2H), 1.02 (m, 4H), 0.69 (t, 3H, *J* = 6.7 Hz). LC-MS (APCI⁺) *m*/*z* 302.1 (MH⁺).

**6-Methyl-4-oxo-l-pentyl-1,4-dihydropyridine-3-carboxylic acid (32).** To a stirred solution of compound **31** (0.2 g; 1.1 mmol) in anhydrous EtOH (10 mL) were added under nitrogen atmosphere, potassium *tert*-butoxide (0.17 g; 1.7 mmol) and n-pentylamine (0.25 mL; 2.2 mmol). This mixture was refluxed under nitrogen for 12 h. EtOH was removed under reduced pressure and the residue dissolved in water. The aqueous phase was washed with EtOAc and the resulting solution was carefully acidified with a 1N HCl solution to pH 2 and extracted with EtOAc. The combined organic extracts were washed with H₂O, dried over MgSO₄ and the solvent removed under reduced pressure to afford pure **32** as a white solid (0.18 g, 73%); mp 126°C; ¹H NMR (CDCl₃) δ 16.61 (s, 1H), 8.43 (s, 1H), 6.58 (s, 1H), 3.97 (t, 2H, *J* = 7.9 Hz), 2.45 (s, 3H), 1.80 (m, 2H), 1.38 (m, 4H), 0.93 (t, 3H, *J* = 6.8 Hz). LC-MS (APCI⁺) *m*/*z* 224.2 (MH⁺).

**5-iodo-6-methyl-4-oxo-1-pentyl-1,4-dihydropyridine-3-carboxylic acid (34).** Carboxylic acid **32** (0.5 g, 2.2 mmol), calcium carbonate (0.45 g, 2.7 mmol) and iodide monochloride (0.3 mL, 4.5 mmol) were dissolved in DMF (20 mL) and stirred at 50 °C during 12 h. The solvent was removed under reduced pressure and the residue taken up in water and extracted with CH₂Cl₂. The organic phase was washed, with water and brine. The organic extract was dried over MgSO₄ and concentrated in vacuo to an orange oil. The crude material was purified by TLC using the appropriate eluent (dichloromethane/methanol 95:5) to leave compound 34 as a yellow solid (0.730 g, 95; mp 154°C; ¹H NMR (CDCl₃) δ 16.58 (s, 1H), 8.40 (s, 1H), 4.02 (t, 2H, *J* = 7.9 Hz), 2.77 (s, 3H), 1.84 (m, 2H), 1.41 (m, 4H), 0.93 (t, 3H, *J* = 6.8 Hz). LC-MS (APCI⁺) *m*/*z* 350.0 (MH⁺).

### Example 5: Characteristics of compounds 6-26.

**(R,S)-*N*3-(1-(1-Adamantyl)ethyl)-1-ethyl-4-oxo-6-phenyl-1,4-dihydropyridine-3-carboxamide (compound 6):** Beige solid (54%); mp 224°C; ¹H NMR (CDCl₃) δ 10.41 (d, 1H, *J* = 9.1 Hz), 8.58 (s, 1H), 7.53 (m, 3H), 7.35 (m, 2H), 6.47 (s, 1H), 3.87 (m, 3H), 2.00 (m, 3H), 1.64 (m, 12H), 1.25 (t, 3H, *J* = 7.3 Hz), 1.13 (d, 3H, *J* = 7.0 Hz). LC-MS (APCI⁺) *m*/*z* 405.1 (MH⁺).

**(R,S)-*N*3-(1-(1-Adamantyl)ethyl)-4-oxo-1-pentyl-6-phenyl-1,4-dihydropyridine-3-carboxamide (compound 7):** White solid (52%); mp 169°C; ¹H NMR (CDCl₃) δ 10.41 (d, 1H, *J* = 9.1 Hz), 8.55 (s, 1H), 7.52 (m, 3H), 7.33 (m, 2H), 6.47 (s, 1H), 3.91 (m, 1H), 3.79 (t, 2H, *J* = 7.7 Hz), 2.00 (m, 3H), 1.64 (m, 14H), 1.15 (m, 7H), 0.79 (t, 3H). LC-MS (APCI⁺) *m*/*z* 447.4 (MH⁺).

**(R,S)-*N*3-(1-(1-Adamantyl)ethyl)-1,6-diphenyl-4-oxo-1,4-dihydropyridine-3-carboxamide (compound 8):** White solid (47%); mp >250°C; ¹H NMR (CDCl₃) δ 10.35 (d, 1H,), 8.70 (s, 1H), 7.32 (m, 6H), 7.10 (m, 4H), 6.86 (s, 1H), 3.90 (m, 1H), 2.00 (m, 3H), 1.69 (m, 12H), 1.17 (d, 3H, *J* = 6.7 Hz). LC-MS (APCI⁺) *m*/*z* 453.4 (MH⁺).

***N*3-((1-Adamantyl)methyl)-4-oxo-1-pentyl-6-phenyl-1,4-dihydropyridine-3-carboxamide (compound 9):** White solid (57%); mp 154°C; ¹H NMR (CDCl₃) δ 10.39 (m, 1H), 8.56 (s, 1H), 7.51 (m, 3H), 7.35 (m, 2H), 6.47 (s, 1H), 3.79 (t, 2H, *J =* 7.7 Hz), 3.16 (d, 2H, *J* = 6.1 Hz), 1.99 (m, 3H), 1.69-1.61 (m, 14H), 1.12 (m, 4H), 0.78 (t, 3H, *J* = 6.9 Hz). LC-MS (APCI⁺) *m*/*z* 433.3 (MH⁺).

***N*3-(1-(3,5-Dimethyl)adamantyl)-4-oxo-1-pentyl-6-phenyl-1,4-dihydropyridine-3-carboxamide (compound 10):** White solid (62%); mp 133°C; ¹H NMR (CDCl₃) δ 10.21 (s, 1H), 8.5 (s, 1H), 7.5-7.35 (m, 5H), 6.45 (s, 1H), 3.77 (t, 2H), 2.00-1.18 (m, 19H), 0.88 (s, 6H), 0.79 (t, 3H, *J* = 7.0 Hz). LC-MS (APCI⁺) *m*/*z* 447.3 (MH⁺).

***N*3-(1-Adamantyl)-1-butyl-4-oxo-6-phenyl-1,4-dihydropyridine-3-carboxamide (compound 11):** White solid (30%); mp 146°C; ¹H NMR (DMSO-*d₆*) δ 10.19 (s, 1H), 8.53 (s, 1H), 7.53 (m, 5H), 6.25 (s, 1H), 3.90 (t, 2H, *J =* 7.4 Hz), 2.03 (m, 9H), 1.66 (m, 6H), 1.40 (m, 2H), 1.04 (m, 2H), 0.65 (t, 3H, *J* = 7.3 Hz). LC-MS (APCI⁺) *m*/*z* 405.2 (MH⁺).

***N*3-(1-Adamantyl)-4-oxo-1-pentyl-6-phenyl-1,4-dihydropyridine-3-carboxamide (compound 12):** Beige solid (55%); mp 145°C; ¹H NMR (CDCl₃) δ 10.17 (s, 1H), 8.51 (s, 1H), 7.51 (m, 3H), 7.34 (m, 2H), 6.45 (s, 1H), 3.77 (t, 2H, *J* = 7.7 Hz), 2.16 (m, 9H), 1.72-1.58 (m, 8H), 1.12 (m, 4H), 0.81 (t, 3H, *J* = 6.9 Hz). LC-MS (APCI⁺) *m*/*z* 419.3 (MH⁺).

***N*3-(1-Adamantyl)-1-hexyl-4-oxo-6-phenyl-1,4-dihydropyridine-3-carboxamide (compound 13):** White solid (32%); mp 167°C; ¹H NMR (DMSO-*d₆*) δ 10.19 (s, 1H), 8.53 (s, 1H), 7.54 (m, 5H), 6.25 (s, 1H), 3.90 (t, 2H, *J =* 7.6 Hz), 2.03 (m, 9H), 1.66 (m, 6H), 1.41 (m, 2H), 1.00 (m, 6H), 0.74 (t, 3H, *J* = 7.0 Hz). LC-MS (APCI⁺) *m*/*z* 433.3 (MH⁺).

***N*3-(Cyclohexyl)-4-oxo-1-pentyl-6-phenyl-1,4-dihydropyridine-3-carboxamide (compound 14):** White solid (64%); mp 120°C; ¹H NMR (CDCl₃) δ 10.31 (d, 1H, *J* = 7.6 Hz), 8.54 (s, 1H), 7.52 (m, 3H), 7.35 (m, 2H), 6.46 (s, 1H), 4.00 (m, 1H), 3.80 (t, 2H, *J* = 7.7 Hz), 1.98-1.12 (m, 16H), 0.79 (t, 3H, *J* = 6.9 Hz). LC-MS (APCI⁺) *m*/*z* 366.2 (MH⁺).

**(R,S)-4-Oxo-1-pentyl-6-phenyl-*N*3-(1-(1,2,3,4-tetrahydronaphthyl))-1,4-dihydropyridine-3-carboxamide (compound 15):** To obtain compound 15, the racemate amine, (R,S)-1,2,3,4-tetrahydro-1-naphthylamine was used. White solid (55%); mp 139°C; ¹H NMR (CDCl₃) δ 10.66 (d, 1H, *J* = 8.2 Hz), 8.61 (s, 1H), 7.52 (m, 3H), 7.35 (m, 3H), 7.13 (m, 3H), 6.44 (s, 1H), 5.45 (m, 1H), 3.81 (t, 2H, *J* = 7.7 Hz), 2.86 (m, 2H), 1.97 (m, 2H), 1.57 (m, 4H), 1.44-1.28 (m, 4H), 0.8 (t, 3H, *J* = 7.0 Hz). LC-MS (APCI⁺) *m*/*z* 415.3 (MH⁺).

**(R)-4-Oxo-1-pentyl-6-phenyl-*N*3-(1-(1,2,3,4-tetrahydronaphthyl))-1,4-dihydropyridine-3-carboxamide (compound 16):** To obtain compound 24, the pure enantiomer, (R)-1,2,3,4-tetrahydro-1-naphthylamine was used. White solid (60%); mp 139°C; ¹H NMR (CDCl₃) and LC-MS (APCI⁺). (See compound 15).

**(S)-4-Oxo-1-pentyl-6-phenyl-*N*3-(1-(1,2,3,4-tetrahydronaphthyl))-1,4-dihydropyridine-3-carboxamide (compound 17):** To obtain compound 25, the pure enantiomer, (S)-1,2,3,4-tetrahydro-1-naphthylamine was used. White solid (52%); mp 139°C; ¹H NMR (CDCl₃) and LC-MS (APCI⁺). (See compound 15).

4-Oxo-1-pentyl-6-phenyl-*N*3-(piperidin-1-yl)-1,4-dihydropyridine-3-carboxamide (compound 18): White powder (40%); mp 122°C; ¹H NMR (CDCl₃) δ 11.17 (s, 1H), 8.56 (s, 1H), 7.53 (m, 3H), 7.33 (m, 2H), 6.48 (s, 1H), 3.81 (t, 2H, *J* = 7.6 Hz), 2.92 (t, 4H, *J* = 5.1 Hz), 1.77-1.48 (m, 8H), 1.12 (m, 4H), 0.79 (t, 3H, *J* = 6.9 Hz). LC-MS (APCI⁺) *m*/*z* 368.3 (MH⁺).

**4-Oxo-1-pentyl-6-phenyl-*N*3-(3(trifluoromethyl)phenyl)-1,4-dihydropyridine-3-carboxamide (compound 19):** White solid (74%); mp 125°C; ¹H NMR (CDCl₃) δ 12.80 (s, 1H), 8.64 (s, 1H), 8.18 (s, 1H), 7.89 (d, 1H, *J* = 7.6 Hz), 7.54-7.37 (m, 7H), 6.56 (s, 1H), 3.86 (t, 2H, *J* = 7.7 Hz), 1.64 (m, 2H), 1.14 (m, 4H), 0.8 (t, 3H, *J* = 6.7 Hz). LC-MS (APCI⁺) *m*/*z* 429.6 (MH⁺).

***N*3-(1-Adamantyl)-6-(4-chlorophenyl)-1-pentyl-4-oxo-1,4-dihydropyridine-3-carboxamide (compound 20):** White powder (28%); mp 190°C; ¹H NMR (DMSO-*d₆*) δ 10.17 (s, 1H), 8.53 (s, 1H), 7.61 (d, 2H, *J* = 8.7 Hz), 7.55 (d, 2H, *J* = 8.7 Hz), 6.27 (s, 1H), 3.9 (t, 2H, *J* = 7.6 Hz), 2.03 (m, 9H), 1.66 (m, 6H), 1.42 (m, 2H), 1.05 (m, 4H), 0.71 (t, 3H, *J* = 6.8 Hz). LC-MS (APCI⁺) *m*/*z* 453.3 (MH⁺).

**6-(4-Chlorophenyl)-*N*3-cyclohexyl-4-oxo-1-pentyl-1,4-dihydropyridine-3-carboxamide (compound 21):** White powder (32%); mp 157°C; ¹H NMR (CDCl₃) δ 10.31 (d, 1H, *J* = 7.9 Hz), 8.58 (s, 1H), 7.61 (d, 2H, *J* = 8.7 Hz), 7.55 (d, 2H, *J* = 8.7 Hz), 6.29 (s, 1H), 3.92 (t, 2H, *J* = 7.6 Hz), 3.82 (m, 1H) 1.83-1.31 (m, 12H), 1.03 (m, 4H), 0.71 (t, 3H, *J* = 6.8 Hz). LC-MS (APCI⁺) *m*/*z* 401.3 (MH⁺).

**6-(4-Chlorophenyl)-4-oxo-1-pentyl-*N*3-(3(trifluoromethyl)phenyl)-1,4-dihydropyridine-3-carboxamide (compound 22):** White powder (38%); mp 100°C; ¹H NMR (CDCl₃) δ 13.03 (s, 1H), 8.80 (s, 1H), 8.29 (s, 1H), 7.78 (d, 2H, *J* = 8.1 Hz), 7.63 (m, 3H), 7.45 (d, 2H, *J* = 7.6 Hz), 6.46 (s, 1H), 3.99 (t, 2H, *J* = 7.4 Hz), 1.45 (m, 2H), 1.06 (m, 4H), 0.72 (t, 3H, *J* = 6.8 Hz). LC-MS (APCI⁺) *m*/*z* 463.2 (MH⁺).

***N*3-(1-Adamantyl)-6-*tert*-butyl-1-pentyl-4-oxo-1,4-dihydropyridine-3-carboxamide (compound 23):** Beige solid (49%); mp 72°C; ¹H NMR (CDCl₃) δ 10.07 (s, 1H), 8.39 (s, 1H), 6.58 (s, 1H), 4.06 (t, 2H, *J* = 8.3 Hz), 2.14 (m, 9H), 1.71 (m, 8H), 1.42 (s, 9H), 1.36 (m, 4H), 0.93 (t, 3H, *J* = 6.6 Hz) . LC-MS (APCI⁺) *m*/*z* 399.3 (MH⁺).

***N*3-(Cyclohexyl)-6-*tert*-butyl-1-pentyl-4-oxo-1,4-dihydropyridine-3-carboxamide (compound 24):** Beige solid (53%); mp 91°C; ¹H NMR (CDCl₃) δ 10.2 (d, 1H, *J* = 7.3 Hz), 8.40 (s, 1H), 6.58 (s, 1H), 4.08 (t, 2H, *J* = 8.3 Hz), 3.96 (m, 1H), 1.94-1.73 (m, 7H), 1.43-1.37 (m, 18H), 0.93 (t, 3H, *J* = 6.6 Hz). LC-MS (APCI⁺) *m*/*z* 347.2 (MH⁺).

***N*3-(1-Adamantyl)-1-pentyl-6-phenyl-4-thioxo-1,4-dihydropyridine-3-carboxamide (compound 25):** Yellow solid (56%); mp 163°C; ¹H NMR (CDCl₃) δ 11.41 (s, 1H), 8.75 (s, 1H), 7.60 (s, 1H), 7.54 (m, 3H), 7.34 (m, 2H), 3.88 (t, 2H, *J* = 7.7 Hz), 2.22 (m, 9H), 1.75-1.56 (m, 8H), 1.13 (m, 4H), 0.79 (t, 3H, *J* = 7.0 Hz). LC-MS (APCI⁺) *m*/*z* 435.3 (MH⁺).

***N*3-(Cyclohexyl)-1-pentyl-6-phenyl-4-thioxo-1,4-dihydropyridine-3-carboxamide (compound 26):** Yellow solid (59%); mp 119°C; ¹H NMR (CDCl₃) δ 11.53 (d, 1H, *J* = 7.3 Hz), 8.76 (s, 1H), 7.60 (s, 1H), 7.53 (m, 3H), 7.35 (m, 2H), 4.09 (m, 1H), 3.91 (t, 2H, *J* = 7.7 Hz), 1.97 (m, 2H), 1.80-1.59 (m, 10H), 1.13 (m, 4H), 0.82 (t, 3H, *J* = 6.8 Hz). LC-MS (APCI⁺) *m*/*z* 383.3 (MH⁺).

### Example 6: Characteristics of compounds 27-30

***Tert*-butyl 4-oxo-1-pentyl-6-phenyl-1,4-dihydropyridin-3-yl carbamate (compound 27):** ¹H NMR (DMSO-*d₆*) δ 8.39 (s, 1H), 7.74 (s, 1H), 7.56-7.43 (m, 5H), 6.12 (s, 1H), 3.81 (t, 2H, *J* = 7.6 Hz), 1.48 (m, 11H), 1.03 (m, 4H), 0.69 (t, 3H, *J* = 6.8 Hz). LC-MS (APCI⁺) *m*/*z* 357.1 (MH⁺).

**5-Amino-1-pentyl-2-phenyl-1*H*-pyridin-4-one (compound 28):** white solid (0.068 g, 95%); mp 98°C; ¹H NMR (DMSO-*d₆*) δ 7.48-7.38 (m, 5H), 7.19 (s, 1H), 5.82 (s, 1H), 4.67 (s, 2H), 3.67 (t, 2H, *J* = 7.3 Hz), 1.46 (m, 2H), 1.04 (m, 4H), 0.72 (t, 3H, *J=* 6.7 Hz). LC-MS (APCI⁺) *mlz* 257.1 (MH⁺).

**1-Cyclohexyl-3-(4-oxo-1-pentyl-6-phenyl-1,4-dihydropyridin-3-yl)urea (compound 29)**: ¹H NMR (DMSO-*d₆*) δ 8.95 (m, 1H), 8.88 (s, 1H), 7.51 (m, 3H) 7.34 (m, 2H), 6.28 (s, 1H), 3.74 (t, 2H, *J* = 7.9 Hz), 3.65 (m, 1H), 3.64 (d, 1H, *J* = 9.9 Hz), 1.6 (m, 7H), 1.44-1.11 (m, 9H), 0.78 (t, 3H, *J=* 7.0 Hz). LC-MS (APCI⁺) *mlz* 382.2 (MH⁺).

***N*-(4-Oxo-1-pentyl-6-phenyl-1,4-dihydropyridin-3-yl)cyclohexanecarboxamide (compound 30):** white solid (38%); mp 179°C; ¹H NMR (CDCl₃) δ 8.94 (s, 1H), 8.57 (s, 1H), 7.48 (m, 3H), 7.33 (m, 2H), 6.37 (s, 1H), 3.72 (t, 2H, *J* = 7.9 Hz), 2.37 (m, 1H), 2.00-1.11 (m, 16H), 0.91 (t, 3H, *J=* 7.0 Hz). LC-MS (APCI⁺) *m*/*z* 367.3 (MH⁺).

### Example 7: Characteristics of compounds 33 and 35-39

***N*3-(1-Adamantyl)-6-methyl-1-pentyl-4-oxo-1,4-dihydropyridine-3-carboxamide (compound 33):** Beige solid (57%); mp 195°C; ¹H NMR (CDCl₃) δ 10.15 (s, 1H), 8.38 (s, 1H), 6.38 (s, 1H), 3.86 (t, 2H, *J* = 7.8 Hz), 2.35 (s, 3H), 2.14 (m, 9H), 1.71 (m, 8H), 1.34 (m, 4H), 0.92 (t, 3H, *J* = 6.9 Hz). LC-MS (APCI⁺) *m*/*z* 357.4 (MH⁺).

***N*3-(1-Adamantyl)-5-iodo-6-methyl-4-oxo-1-pentyl-1,4-dihydropyridine-3-carboxamide (compound 35):** Beige solid (69%); mp 166°C; ¹H NMR (CDCl₃) δ 10.04 (s, 1H), 8.39 (s, 1H), 4.03 (t, 2H, *J* = 7.9 Hz), 2.78 (s, 3H), 2.13 (m, 9H), 1.71 (m, 8H), 1.36 (m, 4H), 0.93 (t, 3H, *J=* 7.0 Hz). LC-MS (APCI⁺) *mlz* 483.2 (MH⁺).

*N***3-(1-Adamantyl)-5-cyclopropyl-6-methyl-4-oxo-1-pentyl-1,4-dihydropyridine-3-carboxamide (compound 36):** white solid (74%); mp 173°C; ¹H NMR (CDCl₃) δ 10.37 (s, 1H), 8.36 (s, 1H), 3.87 (t, 2H, *J* = 7.9 Hz), 2.53 (s, 3H), 2.15 (m, 9H), 1.71 (m, 8H), 1.51 (m, 1H), 1.36 (m, 4H), 1.08 (m, 2H), 0.95 (t, 3H, *J* = 6.7 Hz), 0.54 (m, 2H). LC-MS (APCI⁺) *m*/*z* 397.2 (MH⁺).

***N*3-(1-Adamantyl)-6-methyl-4-oxo-1-pentyl-5-phenyl-1,4-dihydropyridine-3-carboxamide (compound 37):** white solid (92%); mp 208°C; ¹H NMR (CDCl₃) δ 10.19 (s, 1H), 8.50 (s, 1H), 7.42 (m, 3H), 7.16 (m, 2H), 3.95 (t, 2H, *J* = 7.9 Hz), 2.18 (s, 3H), 2.12 (m, 9H), 1.68 (m, 8H), 1.51 (m, 1H), 1.38 (m, 4H), 1.08 (m, 2H), 0.94 (t, 3H, *J=* 7.0 Hz). LC-MS (APCI⁺) *m*/*z* 433.2 (MH⁺).

***N*3-(1-Adamantyl)-6-methyl-4-oxo-1-pentyl-5-*p*-tolyl-1,4-dihydropyridine-3-carboxamide (compound 38):** white solid (87%); mp 190°C; ¹H NMR (CDCl₃) δ 10.24 (s, 1H), 8.48 (s, 1H), 7.23 (d, 2H, *J* = 8.2 Hz), 7.07 (d, 2H, *J* = 8.2 Hz), 3.94 (t, 2H, *J* = 7.9 Hz), 2.38 (s, 3H), 2.18 (s, 3H), 2.12 (m, 9H), 1.68 (m, 8H), 1.38 (m, 4H), 0.94 (t, 3H, *J* = 6.7 Hz). LC-MS (APCI⁺) *m*/*z* 447.3 (MH⁺).

**5-(3-cyanophenyl)-*N*3-(1-Adamantyl)-6-methyl-4-oxo-1-pentyl-1,4-dihydropyridine-3-carboxamide (compound 39):** white solid (80%); mp 69°C; ¹H NMR (CDCl₃) δ 9.99 (s, 1H), 8.52 (s, 1H), 7.68 (d, 1H, *J* = 7.6 Hz), 7.54 (t, 1H, *J* = 7.9 Hz), 7.46 (s, 1H), 7.44 (d, 1H, *J=* 7.9 Hz), 3.96 (t, 2H, *J* = 7.9 Hz), 2.18 (s, 3H), 2.12 (m, 9H), 1.68 (m, 8H), 1.38 (m, 4H), 0.94 (t, 3H, *J* = 6.7 Hz). LC-MS (APCI⁺) *m*/*z* 458.3 (MH⁺).

### Example 8: Biological data

### Competition binding assays

hCB1 and hCB2 membranes were purchased from PerkinElmer (Belgium). [³H]-SR141716A (52 Ci/mol) was purchased from Amersham (Roosendaal, The Netherlands) and [³H]-CP-55,940 (101 Ci/mol) was from NEN Life Science (Zaventem, Belgium). Glass fiber filters were purchased from Whatman (Maidstone, U.K.), while Aqualuma was from PerkinElmer (Schaesberg, The Netherlands). Stock solutions of the compounds were prepared in DMSO and further diluted (100x) with the binding buffer to the desired concentration. Final DMSO concentrations in the assay were less than 0.1 %.

Under these conditions, using [³H]-SR141716A, the *Bₘₐₓ* value was 57 pmoles/mg protein and K_{d} value was 1.13 (0.13 nM) for the *h*CB1 cannabinoid receptor. Using [³H]-CP-55,940, the *Bₘₐₓ* value was 57 pmoles/mg protein and K_{d} value was 4.3 (0.13 nM) for the hCB2 cannabinoid receptor

The competitive binding experiments were performed using [³H]-SR141716A (1 nM) or [³H]-CP-55,940 (1nM) as radioligands for the *h*CB1 and the *h*CB2 cannabinoid receptor, respectively, at 30°C in plastic tubes, and 40 µg of membranes per tube resuspended in 0.5 mL (final volume) of binding buffer (50 mM Tris-HCl, 3 mM MgCl₂, 1 mM EDTA, 0.5 % bovine serum albumine, pH 7.4). The test compounds were present at varying concentrations and the non-specific binding was determined in the presence of 10 µM HU-210. After 1 h the incubation was stopped and the solutions were rapidly filtered through 0.5% PEI pretreated GF/B glass fiber filters on a M-48T Brandell cell harvester and washed twice with 5 mL ice-cold binding buffer without serum albumin. The radioactivity on the filters was measured in a Pharmacia Wallac 1410 β-counter using 10 mL of Aqualuma, after 10 s shaking and 3 h resting. Assays were performed at least in triplicate.

### [³⁵S]-GTPγS binding assays.

[³⁵S]-GTPγS (1173 Ci/mmol) was purchased from Amersham (Roosendaal, The Netherlands). The binding experiments were performed at 30°C in plastic tubes containing 40 µg protein in 0.5 mL (final volume) of binding buffer (50 mM Tris-HCl, 3 mM MgCl₂, 1 mM EDTA, 100 mM NaCl, 0.1 % bovine serum albumin, pH 7.4) supplemented with 20 µM GDP.

The assay was initiated by the addition of [³⁵S]-GTPγS (0.05 nM, final concentration). The tubes were incubated for 1 h. The incubations were terminated by the addition of 5 mL ice-cold washing buffer (50 mM Tris-HCl, 3 mM MgCl₂, 1 mM EDTA, 100 mM NaCl). The suspension was immediately filtered through GF/B filters using a 48-well Brandell cell harvester and washed twice with the same ice-cold buffer. The radioactivity on the filters was counted as mentioned above. Assays were performed in triplicate.

### Data analysis.

IC₅₀ and EC₅₀ values were determined by non-linear regression analysis performed using the GraphPad prism 4.0 program (GraphPad Software, San Diego). The Kᵢ values (expressed as pKᵢ) were calculated based on the Cheng-Prusoff equation : Kᵢ = IC₅₀/(1+L/K_{d}). Statistical signification of [³⁵S]-GTPγS assay results was assessed using an one-way ANOVA followed by a Dunett post-test.

### Results

Compounds of the invention may bind to the CB2 receptor with greater affinity that the CB1 receptor, with selectivity indexes up to 150. Such compounds may be particularly useful in treating CB2 receptor mediated diseases.

The compounds of the present invention typically show binding activities of > 60% at 10 µM concentrations. Compounds **6, 7, 9-21, 23-27, 30, 33** and 35 exhibit binding affinities (Ki) at the CB2 receptor of less than 500 nM.

The compounds were tested in the CB2 functional activity assay. Compounds **7, 9-22** and **30** were functional inverse agonist (Eₘₐₓ < 62%) and compounds **23, 24** and **33** were found agonist (Eₘₐₓ > 135%).

**Table V: Functionality of compounds of the formula I**

| | | **Functionality CB2** | |
|---|---|---|---|
| **Compound** | **Kᵢ CB2** | **EC₅₀ (nM)** | **E_{Max}(%)** |
| **No.** | **(nM)** | | |
| **6** | 414 ± 61 | ND | ND |
| **7** | 14.3 ± 2,1 | 6.5 ± 1.5 | 46 ± 3 |
| **8** | >3000 | ND | ND |
| **9** | 23.3 ± 1.9 | 10.9 ± 2.8 | 37 ± 1 |
| **10** | 6.6 ± 0.7 | 5.8 ± 1.1 | 41 ± 2 |
| **11** | 18.4 ± 1.3 | 7.4 ± 0.9 | 40 ± 2 |
| **12** | 4.0 ± 0.4 | 3.2± 1.6 | 39 ± 2 |
| **13** | 13.5 ± 0.9 | 8.7 ± 0.9 | 33 ± 1 |
| **14** | 10.6 ± 1.1 | 1.9 ± 0.5 | 62 ± 2 |
| **15** | 30.9 ± 3.3 | 5.7 ± 0.7 | 40 ± 1 |
| **16** | 10.1 ± 1.1 | 1.9 ± 0.4 | 42 ± 2 |
| **17** | 369 ± 41 | 60 ± 16 | 48 ± 3 |
| **18** | 92 ± 7 | 26 ± 6 | 48 ± 2 |
| **19** | 25.8 ± 3 | 14 ± 4 | 42 ± 4 |
| **20** | 79 ± 7 | 23 ± 6 | 42 ± 2 |
| **21** | 78 ± 9 | 22 ± 4 | 39 ± 2 |
| **22** | 1100 ± 128 | 158 ± 42 | 42 ± 3 |
| **23** | 29 ± 3 | 12.2 ± 2.5 | 212 ± 3 |
| **24** | 29 ± 3 | 7.8 ± 3.5 | 135 ± 3 |
| **25** | 18.8 ± 2.3 | 17±2 | 30 ± 1 |
| **26** | 48 ± 4 | 27 ± 3 | 45 ± 1 |
| **27** | 209 ± 33 | 164 ± 281 | 26 ± 3 |
| **29** | ND | ND | ND |
| **30** | 51 ± 4 | 25 ± 3 | 43 ± 2 |
| **33** | 20 ± 3 | 5.5 ± 1.1 | 148 ± 2 |
| **35** | 9.0 ± 1.6 | ND | ND |

### Example 9:

C57B16 (Breeding January, Le Genest Saint Isle, France) mice (n=15 per group) had free access to standard mouse chow and tap water. For colitis induction, mice were anesthetized for 90-120 min and received an intrarectal administration of trinitrobenzene sulfonic acid (TNBS) (40 µl, 150 mg/kg) dissolved in a 1:1 mixture of 0.9% NaCl with 100% ethanol. Control mice received a 1:1 mixture of 0.9% NaCl with 100% ethanol or a saline solution using the same technique. Animals were killed 3 d after TNBS administration. JWH-133 (Tocris Bioscience, Bristol, UK) and the new CB2 agonists were administered intraperitoneally once daily, starting 3 d before colitis induction. Body-weight changes, macroscopic and histological indications of colitis were evaluated blindly by two investigators. The colon of each mouse was examined to evaluate the macroscopic lesions according to the Wallace criteria. The Wallace score rates macroscopic lesions on a scale from 0 to 10 based on features reflecting inflammation, such as hyperemia, thickening of the bowel, and extent of ulceration. A colon specimen located precisely 2 cm above the anal canal will be used for histological evaluation according to the Ameho criteria. This grading on a scale from 0 to 6 takes into account the degree of inflammation infiltrate, the presence of erosion, ulceration, or necrosis, and the depth and surface extension of lesions. The other parts of the colon were frozen and used to quantify MPO activity as well as IL-1β and TNF-α mRNA levels.

### REFERENCES

(1) Gaoni Y et al. J. Am. Chem. Soc. 1964, 86, 1646-1647.
(2) Matsuda LA et al. Nature, 1990, 346, 561-4.
(3) Munro S et al. Nature, 1993, 365, 61-5.
(4) Cravatt BF et al. Nature, 1996, 384, 83-7.
(5) Devane WA et al. Science, 1992, 258, 1946-9.
(6) Mechoulam R et al. Biochem. Pharmacol., 1995, 50, 83-90.
(7) Mackie K. et al. Annu. Rev. Pharmacol. Toxicol., 2006, 46, 101-22.
(8) Guindon J et al. Br. J. Pharmacol. 2008, 153, 319-34.
(9) Rousseaux C et al. Nat. Med., 2007, 13, 35-7.
(10) Sanson M et al. Neurogastroenterol. Motil., 2006, 18, 949-956.
(11) Galiègue S et al. Eur. J Biochem., 1995, 232, 54-61.
(12) Wright K et al. Gastroenterology, 2005, 129, 437-453.
(13) Buckley NE et al. Eur. J. Pharmacol., 2000, 396, 141-149.
(14) Ihenetu K et al. Eur. J. Pharmacol., 2003, 458, 207-215.
(15) Miller AM et al. Br. J. Pharmacol., 2008, 153, 299-308.
(16) Wright KL et al. Br. J. Pharmacol., 2008, 153, 263-270.
(17) Kilbourn EE et al. J. Org. Chem., 1972, 37, 1145-8.
(18) Bassini C et al. Il Farmaco, 1993, 48, 159-89.
(19) Stem E et al. J Med Chem. 2006, 49, 70-9.
(20) Stem E et al. J Med Chem. 2007, 50, 5471-84.
(21) McCombie SW et al. J Org. Chem., 1991, 56, 4963-7.
(22) Bassini C et al. Il Farmaco, 1993, 48, 159-89.
(23) Blackaby WP et al. Bioorg Med Chem Lett. 2005 , 15, 4998-5002.
(24) Wallace DJ et al. Tetrahedron Lett. 2002, 43, 6987-6990.

## Claims

1. Compounds of formula (I) below: 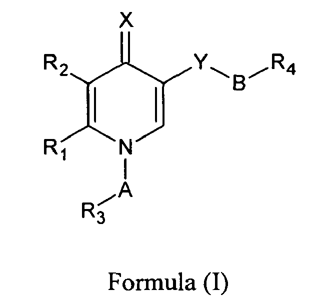 or pharmaceutically acceptable salts or solvates thereof, wherein:
X represents oxygen or sulphur;
Y represents C=O, CONH, NHCO, NHCONH, NHSO₂-CO, NH(CO)O, NR₉; R₉ representing a hydrogen atom or C₁₋₃ alkyl;
A represents (CR₇R₈)ₘ; R₇ and R₈, identical or different, representing an hydrogen atom or C₁₋₃ alkyl; and m representing an integer equal to 0, 1, 2, 3 or 4; and when m=0, A represents a single bond;
B represents (CR₇R₈)ₙ; R₇ and R₈ being as defined above and n representing an integer equal to 0, 1, 2, 3 or 4; and when n=0, B represents a single bond;
R₁ and R₂ are identical or different and represent independently a hydrogen atom, a halogen atom, CN, CF₃, OCF₃, OCHF₂, an alkyl, an alkoxy, (CH₃)₃C, a cycloalkyl, an optionally substituted 5 to 10 membered aryl, an optionally substituted 5 to 7 membered monocyclic heteroaryl containing 1 to 3 heteroatoms selected from O, N, S, or an optionally substituted 9 to 10 membered fused bicyclic heteroaryl containing 1 to 3 heteroatoms selected from O, N and S; and
R₃ and R₄ are identical or different and represent independently a hydrogen atom, an alkyl, an alkoxy, an optionally substituted tetrahydropyranyl, morpholinyl or cycloalkyl, an optionally substituted 6 to 10 membered aryl, an optionally substituted 5 to 7 membered monocyclic heteroaryl containing 1 to 3 heteroatoms selected from O, N, S, or an optionally substituted 9 to 10 membered fused bicyclic heteroaryl containing 1 to 3 heteroatoms selected from O, N and S.

2. Compound of claim 1, **characterized in that** when R₁ or R₂ represent an optionally substituted 5 to 10 membered aryl, an optionally substituted 5 to 7 membered monocyclic heteroaryl containing 1 to 3 heteroatoms selected from O, N, S, or an optionally substituted 9 to 10 membered fused bicyclic heteroaryl containing 1 to 3 heteroatoms selected from O, N and S, they may be substituted by one or more substituents which may be the same or different, selected from the group consisting of an halogen, hydroxyl, CN, nitro, NR₇R₈, CONR₇R₈, CF₃, OCF₃, OCHF₂, an alkyl, an alkoxy, COC₁₋₆ alkyl, COC₁₋₆ alkoxy, NHCOC₁₋₆ alkyl and COOH.

3. Compound of any of claims 1 or 2, **characterized in that** when R₃ or R₄, represent an optionally substituted tetrahydropyranyl, morpholinyl, cycloalkyl, they may be substituted by one or more substituents which may be, identical or different, selected from the group consisting of halogen, hydroxyl, CN, nitro, NR₇R₈, CONR₇R₈, CF₃, OCF₃, OCHF₂, alkyl, alkoxy, COC₁₋₄ alkyl, COC₁₋₄ alkoxy, NHCOC₁₋₆ alkyl and COOH.

4. Compound of any of claims 1 or 2, **characterized in that** when R₃ or R₄, represent an optionally substituted 6 to 10 membered aryl, an optionally substituted 5 to 7 membered monocyclic heteroaryl containing 1 to 3 heteroatoms selected from O, N, S, or an optionally substituted 9 to 10 membered fused bicyclic heteroaryl containing 1 to 3 heteroatoms selected from O, N and S, they may be substituted by one or more substituents, which may be identical or different, selected from the group consisting of an halogen, hydroxyl, CN, nitro, NR₇R₈, CONR₇R₈, CF₃, OCF₃, OCHF₂, an alkyl, an alkoxy, COC₁₋₆ alkyl, COC₁₋₆ alkoxy, NHCOC₁₋₆ alkyl and COOH.

5. Compound of any of claims 1 to 4, **characterized in that** the compounds of formula (I) have a binding affinity to the CB2 receptor, measured by the inhibition constant Kᵢ = IC₅₀/(1+L/K_{d}), said Kᵢ being of less than 1500 nM, preferably of less than 500 nM and more preferably of less than 100 nM.

6. Compound of claim 5, **characterized in that** in formula I
- m=0, A represents a single bond and R₃ represents an alkyl having 1 to 6 carbon atoms, more preferably butyl, pentyl or hexyl; and/or
- the radical R₂ represents preferably an hydrogen atom, a halogen atom, an alkyl having 1 to 6 carbon atoms, a cycloalkyl or an aryl, more preferably an hydrogen atom, a iodine atom, cyclopropyl *p*-tolyl, meta-cyanophenyl or phenyl; and/or
- n=0; B represents a single bond and R₄ represents an alkyl, a cycloalkyl, an aryl or an heteroaryl, more preferably t-butyl, cyclohexyl, adamantyl, adamantylethyl, dimethyladamantyl or phenyl, trifluoromethylphenyl, tetrahydronaphtyl or piperidin and the radical Y represents CONH, NHCO, NHCONH or NH(CO)O.

7. Compound of claim 6, **characterized in that** the radical R₁ represents preferably an alkyl having 1 to 6 carbon atoms, more preferably methyl or t-butyl, preferably, said compounds of the formula (I) are chosen from the group consisting of: *N*3-(1-Adamantyl)-6-*tert*-butyl-1-pentyl-4-oxo-1,4-dihydropyridine-3-carboxamide (**Compound 23**); *N*3-(Cyclohexyl)-6-*tert*-butyl-1-pentyl-4-oxo-1,4-dihydropyridine-3-carboxamide **(Compound 24)** and *N*3-(1-Adamantyl)-6-methyl-1-pentyl-4-oxo-1,4-dihydropyridine-3-carboxamide **(Compound 33)** and are agonists of the CB2 receptor.

8. Compound of claim 6, **characterized in that** radical R₁ represents preferably an aryl, more preferably a phenyl or a 4-Cl-phenyl; preferably, said compounds of the formula (I) are chosen from the group consisting of: (R,S)-*N*3-(1-(1-Adamantyl)ethyl)-4-oxo-1-pentyl-6-phenyl-1,4-dihydropyridine-3-carboxamide **(Compound 7),** *N*3-((1-Adamantyl)methyl)-4-oxo-1-pentyl-6-phenyl-1,4-dihydropyridine-3-carboxamide **(Compound 9),** N3-(1-(3,5-Dimethyl)adamantyl)-4-oxo-1-pentyl-6-phenyl-1,4-dihydropyridine-3-carboxamide **(Compound 10),** *N*3-(1-Adamantyl)-1-butyl-4-oxo-6-phenyl-1,4-dihydropyridine-3-carboxamide (**Compound 11**), *N*3-(1-Adamantyl)-4-oxo-1-pentyl-6-phenyl-1,4-dihydropyridine-3-carboxamide **(Compound 12),** *N*3-(1-Adamantyl)-1-hexyl-4-oxo-6-phenyl-1,4-dihydropyridine-3-carboxamide **(Compound 13),** *N*3-(Cyclohexyl)-4-oxo-1-pentyl-6-phenyl-1,4-dihydropyridine-3-carboxamide **(Compound 14),** (R,S)-4-Oxo-1-pentyl-6-phenyl-*N*3-(1-(1,2,3,4-tetrahydronaphthyl))-1,4-dihydropyridine-3-carboxamide **(Compound 15),** (R)-4-Oxo-1-pentyl-6-phenyl-*N*3-(1-(1,2,3,4-tetrahydronaphthyl))-1,4-dihydropyridine-3-carboxamide **(Compound 16),** (S)-4-Oxo-1-pentyl-6-phenyl-*N*3-(1-(1,2,3,4-tetrahydronaphthyl))-1,4-dihydropyridine-3-carboxamide **(Compound 17),** 4-Oxo-1-pentyl-6-phenyl-*N*3-(piperidin-1-yl)-1,4-dihydropyridine-3-carboxamide **(Compound 18),** 4-Oxo-1-pentyl-6-phenyl-*N*3-(3(trifluoromethyl)phenyl)-1,4-dihydropyridine-3-carboxamide **(Compound 19),** *N*3-(1-Adamantyl)-6-(4-chlorophenyl)-1-pentyl-4-oxo-1,4-dihydropyridine-3-carboxamide **(Compound 20),** 6-(4-Chlorophenyl)-*N*3-cyclohexyl-4-oxo-1-pentyl-1,4-dihydropyridine-3-carboxamide **(Compound 21),** 6-(4-Chlorophenyl)-4-oxo-1-pentyl-*N*3-(3(trifluoromethyl)phenyl)-1,4-dihydropyridine-3-carboxamide **(Compound 22),** *N*3-(1-Adamantyl)-1-pentyl-6-phenyl-4-thioxo-1,4-dihydropyridine-3-carboxamide **(Compound 25),** *N*3-(Cyclohexyl)-1-pentyl-6-phenyl-4-thioxo-1,4-dihydropyridine-3-carboxamide **(Compound 26),** *Tert*-butyl 4-oxo-1-pentyl-6-phenyl-1,4-dihydropyridin-3-yl carbamate **(Compound 27),** 5-Amino-1-pentyl-2-phenyl-1*H-*pyridin-4-one **(Compound 28)** and *N*-(4-Oxo-1-pentyl-6-phenyl-1,4-dihydropyridin-3-yl)cyclohexanecarboxamide **(Compound 30)** and are inverse agonists of the CB2 receptor.

9. Process of preparation of a compound of the formula (I) wherein R₂ represents a hydrogen atom and Y represents CONH comprising the following steps:
(i) condensation of ethyl acetoacetate with N,N-dimethylformamide
/ dimethylsulfate adduct and triethylamine,
(ii) addition of the product of step (i) on the appropriate acyl chloride with subsequent cyclisation,
(iii) aminolysis of the product of step (ii),
(iv) facultatively thionation of the product of step (iii),
(v) saponification of the ethyl functions of the products of steps (iii) or (iv), and
(vi) amidation of the product of step (v).

10. Process of preparation of a compound of the formula (I) wherein R₂ represents a hydrogen atom, Y represents NHCO and R₃ represents C₅H₁₁, said process comprising steps i) to (v) as specified in claim 9and further on the following steps:
vi) Curtius rearrangement of carboxylic acid into carbamate,
(vii) deprotection (Boc proctective group) of the product of step (i) into amine, and
(viii) coupling reaction of the product of step (ii) into urea or amide with a isocyanate or a carboxylic acid derivative.

11. Process of preparation of a compound of the formula (I) wherein R₁ represents an alkyl, preferably a methyl, R₂ represents a hydrogen atom, n=0, R₃ represents anlakyl, preferably C₅H₁₁, Y represents CONH, m=0 and R₄ represents an adamantyl having the following steps:
(i) condensation of 4-hydroxy-6-methyl-2-pyrone with dimethylformamide-dimethyl acetal (DMF-DMA),
(ii) aminolysis of the product of step (i),
(iii) coupling reaction of the product of step (ii) into amide with a carboxylic acid derivative.

12. Process of preparation of a compound of the formula (I) wherein R₁ represents an alkyl, preferably a methyl, R₂ represents a iodine atom, n=0, R₃ represents an alkyl, preferably C₅H₁₁, Y represents CONH, m=0 and R₄ represents an adamantyl having the following steps:
(i) condensation of 4-hydroxy-6-methyl-2-pyrone with dimethylformamide-dimethyl acetal (DMF-DMA),
(ii) aminolysis of the product of step (i),
(iii) iodination of the product of step (ii) and
(iv) coupling reaction of the product of step iii) into amide with a carboxylic acid derivative.

13. Process of preparation of compounds of the formula (I) wherein R₁ represents an alkyl, preferably a methyl, R₂ a cyclopropyl, a phenyl, a para-tolyl or a meta-cyanophenyl, n=0, R₃ represents an alkyl, preferably C₅H₁₁, Y represents CONH, m=0 and R₄ represents an adamantyl, having the following steps:
(i) condensation of 4-hydroxy-6-methyl-2-pyrone with dimethylformamide-dimethyl acetal (DMF-DMA),
(ii) aminolysis of the product of step (i),
(iii) iodination of the product of step (ii),
(iv) coupling reaction of the product of step iii) into amide with a carboxylic acid derivative (step iii in scheme 3) and
(v) Suzuki cross-coupling reaction.

14. Pharmaceutical composition comprising a compound of any of claims 1 to 8 or obtained by the process of claims 9 to 13 and at least a pharmaceutically acceptable vehicle.

15. Compounds of the formula (I) of claims 1 to 8 as drugs.

16. Compounds of formula (I) of claims 1 to 8 for use in the treatment of conditions which are mediated by the activity of CB2 receptor.

17. Use of a compound of formula (I) or a pharmaceutically salt or solvate thereof of claims 1 to 8, for the manufacture of a therapeutic agent for the treatment or prevention of a condition such as an immune disorder, an inflammatory disorder, pain, rheumatoid arthritis, multiple sclerosis, inflammatory bowel diseases, osteoarthritis or osteoporosis, an neurological disease.
